(19) Europäisches Patentamt
European Patent Office
Office européen des brevets

(11) Veröffentlichungsnummer: **0 159 290**
**A1**

(12) # EUROPÄISCHE PATENTANMELDUNG

(21) Anmeldenummer: 85810125.6

(22) Anmeldetag: 22.03.85

(51) Int. Cl.⁴: **A 01 N 25/32**

(30) Priorität: 28.03.84 CH 1552/84

(43) Veröffentlichungstag der Anmeldung: 23.10.85 Patentblatt 85/43

(84) Benannte Vertragsstaaten: AT BE CH DE FR GB IT LI NL SE

(71) Anmelder: CIBA-GEIGY AG
Postfach
CH-4002 Basel(CH)

(72) Erfinder: Hubele, Adolf, Dr.
Obere Egg 9
CH-4312 Magden(CH)

(72) Erfinder: Nyffeler, Andreas, Dr.
Gründlerstrasse 4
CH-4312 Magden(CH)

(54) **Verwendung von Chinolinderivaten zum Schützen von Kulturpflanzen.**

(57) Die Verwendung von Chinolinderivaten der Formel

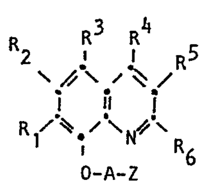

worin $R_1$, $R_2$ und $R_3$ unabhängig voneinander Wasserstoff, Halogen, Nitro, Cyano, $C_1$-$C_3$-Alkyl oder $C_1$-$C_3$-Alkoxy, $R_4$, $R_5$ und $R_6$ unabhängig voneinander Wasserstoff, Halogen oder $C_1$-$C_3$-Alkyl,
A eine der Gruppen $-CH_2-$, $-CH_2CH_2-$ oder $-CH(CH_3)-$ und
Z a) Cyan oder Amidoxim, welches am Sauerstoffatom acyliert sein kann, oder
b) eine Carboxylgruppe oder ein Salz davon, eine Mercaptocarbonylgruppe oder ein Salz davon, eine Carbonsäureestergruppe, eine Carbonsäurethiolestergruppe, eine unsubstituierte oder substituierte Carbonsäureamidgruppen, ein cyclisiertes, unsubstituiertes oder substituiertes Derivat einer Carbonsäureamidgruppe oder eine Carbonsäurehydrazidgruppe, oder
A und Z zusammen einen unsubstituierten oder substituierten Tetrahydrofuran-2-on-Ring
bedeuten, unter Einschluss ihrer Säureadditionssalze und Metallkomplexe, zum Schützen von Kulturpflanzen gegen schädigende Wirkungen von herbizid wirksamen Derivaten von 2-[4-(Benzoxazol-, Benzothiazol- und Chinoxalin-2-yl-oxy)-phenoxy]-propionsäure-estern.
Die vorgenannten herbiziden Derivate entsprechen der Formel II

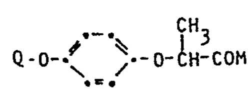 (II),

worin M für $-OR'$ oder $-N(CN)-R_2'$, R' für $C_1$-$C_{12}$-Alkyl, welches durch 1 bis 6 Halogenatome, durch $C_1$-$C_6$-Alkoxy oder $C_1$-$C_4$-Alkylthio substituiert ist, $R_2'$ für $C_1$-$C_4$-Alkyl, $C_3$-$C_4$-Alkenyl, $C_3$-$C_4$-Alkinyl oder Alkoxyalkyl mit insgesamt 2 bis 4 Kohlenstoffatomen, und Q für

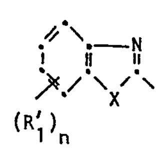 oder 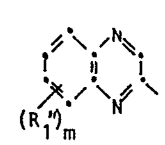

./...

EP 0 159 290 A1

Croydon Printing Company Ltd.

worin X ein Sauerstoff- oder Schwefelatom, $R_1'$ und $R_1''$ Halogen, Trifluormethyl, Nitro, Cyano, $C_1$-$C_4$-Alkyl oder $C_1$-$C_4$-Alkoxy und n und m 0, 1 oder 2 bedeuten, oder, wenn M $-N(CN)-R_2'$ bedeutet, zusätzlich für

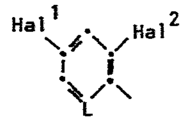

,

worin $Hal^1$ Fluor, Chlor, Brom, Jod oder Trifluormethyl, $Hal^2$ Fluor, Chlor, Brom, Trifluormethyl oder Wasserstoff und L das Strukturelement $=N-$ oder $=CH-$ bedeuten, steht.

0159290

CIBA-GEIGY AG
Basel (Schweiz)

5-14814/+

# Verwendung von Chinolinderivaten zum Schützen von Kulturpflanzen

Die vorliegende Erfindung betrifft die Verwendung von Chinolinderivaten zum Schützen von Kulturpflanzen gegen schädigende Wirkungen herbizid wirksamer Derivate von 2-[4-(Benzoxazol-, Benzthiazol- und Chinoxalin-2-yl-oxy)-phenoxy]-propionsäure-estern.

Beim Einsatz von Herbiziden wie beispielsweise den vorstehend genannten Imidazol-Derivaten können in Abhängigkeit von Faktoren wie beispielsweise Dosis des Herbizids und Applikationsart, Art der Kulturpflanze, Bodenbeschaffenheit und klimatischen Bedingungen, wie beispielsweise Belichtungsdauer, Temperatur und Niederschlagsmengen, die Kulturpflanzen in erheblichem Masse geschädigt werden. Insbesondere kann es zu starken Schädigungen kommen, wenn im Rahmen der Fruchtfolge nach Kulturpflanzen, die gegen die Herbizide resistent sind, andere Kulturpflanzen angebaut werden, welche keine oder nur unzureichende Resistenz gegenüber den Herbiziden aufweisen.

Es ist aus den europäischen Patentpublikationen 86 750 und 94 349 bekannt, dass sich Chinolinderivate zum Schützen von Kulturpflanzen gegen schädigende Wirkungen aggressiver Agrarchemikalien einsetzen lassen.

Es wurde nun gefunden, dass überraschenderweise ein Schutz von Kulturpflanzen gegen Schäden, welche durch herbizid wirksame Derivate von 2-[4-(Benzoxazol-, Benzthiazol- und Chinoxalin-2-yl-oxy)-phenoxy]-propionsäure-estern verursacht werden, durch Behandlung der Kulturpflanzen, von Teilen dieser Pflanzen oder von für

den Anbau der Kulturpflanzen bestimmten Böden mit einem Safener aus einer Gruppe von Chinolinderivaten erzielt werden kann. Die herbizide Wirkung gegenüber Unkräutern und Ungräsern wird durch die Chinolinderivate nicht aufgehoben.

Chinolinderivate, welche zum Schützen von Kulturpflanzen vor schädigenden Wirkungen herbizid wirksamer Derivate von 2-[4-(Benzoxazol-, Benzthiazol- und Chinoxalin-2-yl-oxy)-phenoxy]-propionsäure-estern geeignet sind, entsprechen der Formel I

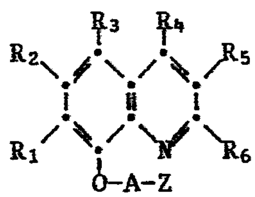

(I),

worin $R_1$, $R_2$ und $R_3$ unabhängig voneinander Wasserstoff, Halogen, Nitro, Cyano, $C_1$-$C_3$-Alkyl oder $C_1$-$C_3$-Alkoxy,
$R_4$, $R_5$ und $R_6$ unabhängig voneinander Wasserstoff, Halogen oder $C_1$-$C_3$-Alkyl,
A eine der Gruppen $-CH_2-$, $-CH_2-CH_2-$ oder $-CH(CH_3)-$ und
Z a) Cyan oder Amidoxim, welches am Sauerstoffatom acyliert sein kann, oder
b) eine Carboxylgruppe oder ein Salz davon, eine Mercaptocarbonylgruppe oder ein Salz davon, eine Carbonsäureestergruppe, eine Carbonsäurethiolestergruppe, eine unsubstituierte oder substituierte Carbonsäureamidgruppe, ein cyclisiertes, unsubstituiertes oder substituiertes Derivat einer Carbonsäureamidgruppe oder eine Carbonsäurehydrazidgruppe, oder
A und Z zusammen einen unsubstituierten oder substituierten Tetrahydrofuran-2-on-Ring
bedeuten, unter Einschluss ihrer Säureadditionssalze und Metallkomplexe.

Unter Amidoxim ist die Gruppe 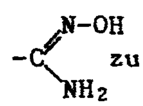 zu verstehen. Das Amidoxim kann

am Sauerstoffatom acyliert sein. Als am Sauerstoffatom acylierte Amidoxime kommen solche der Formel

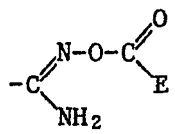

in Betracht, in denen E für $-R_7$, $-OR_8$, $-SR_9$ oder $-NR_{10}R_{11}$ steht, wobei

$R_7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl substituiert ist, Benzyl, welches unsubstituiert oder durch Halogen, Nitro oder $C_1$-$C_3$-Alkyl substituiert ist, oder einen 5- bis 6-gliedrigen heterocyclischen Ring, welcher ein oder zwei Heteroatome aus der Gruppe N, O oder S enthält und unsubstituiert oder durch Halogen substituiert ist,

$R_8$, $R_9$ und $R_{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist,

$R_{11}$ Wasserstoff, $C_1$-$C_8$-Alkyl oder $C_1$-$C_3$-Alkoxy, oder

$R_{10}$ und $R_{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann, bedeuten.

Bei $R_7$ als Heterocyclus kann es sich um gesättigte, teilgesättigte oder ungesättigte Heterocyclen handeln, wie beispielsweise Thiophen, Furan, Tetrahydrofuran und Pyrimidin.

Als Heterocyclen, welche von $R_{10}$ und $R_{11}$ gemeinsam mit dem Stickstoffatom, an das sie gebunden sind, gebildet werden, kommen gesättigte, teilgesättigte oder ungesättigte Heterocyclen in Betracht. Beispiele für solche Heterocyclen sind Pyrrolidin,

Pyrrolin, Pyrrol, Imidazolidin, Imidazolin, Imidazol, Piperazin, Pyridin, Pyrimidin, Pyrazin, Thiazin, Oxazol, Thiazol und insbesondere Piperidin und Morpholin.

Unter Alkyl als Bestandteil des acylierten Amidoxims Z kommen im Rahmen der jeweils angegebenen Anzahl von Kohlenstoffatomen alle geradkettigen und alle verzweigten Alkylgruppen in Betracht.

In der Bedeutung von $R_7$ steht $C_3$-$C_6$-Cycloalkyl für Cyclopropyl, Cyclobutyl, Cyclopentyl und Cyclohexyl.

Von den $C_2$-$C_4$-Alkenyl- und $C_3$-$C_6$-Alkinylgruppen als Bestandteile des acylierten Amidoxims Z sind vor allem Vinyl, Allyl, 1-Propenyl, Isopropenyl und Propinyl zu erwähnen.

Für Z als Carbonsäureestergruppe oder Carbonsäurethiolestergruppe kommt ein entsprechender Säurerest in Betracht, der beispielsweise durch einen gegebenenfalls substituierten, aliphatischen Rest oder einen gegebenenfalls über einen aliphatischen Rest gebundenen und gegebenenfalls substituierten cycloaliphatischen, aromatischen oder heterocyclischen Rest verestert ist.

Als Carbonsäureesterrest bevorzugt ist der Rest $-COOR_{12}$ und als Carbonsäurethiolesterrest bevorzugt ist der Rest $-COSR_{13}$, wobei $R_{12}$ und $R_{13}$ die nachfolgend angegebenen Bedeutungen haben: gegebenenfalls substituierter Alkyl-, Alkenyl-, Alkinyl-, Cycloalkyl-, Phenyl- oder Naphthylrest oder gegebenenfalls substituierter heterocyclischer Rest. Die Reste $-COOR_{12}$ und $-COSR_{13}$ schliessen auch die freien Säuren ein, wobei $R_{12}$ und $R_{13}$ für Wasserstoff stehen, sowie die Salze davon, wobei $R_{12}$ und $R_{13}$ für ein Kation stehen. Als Salzbildner eignen sich hier besonders Metalle und organische Stickstoffbasen, vor allem quaternäre Ammoniumbasen. Hierbei kommen als zur Salzbildung geeignete Metalle Erdalkalimetalle, wie Magnesium oder Calcium, vor allem aber die Alkalimetalle in Betracht, wie Lithium und insbesondere Kalium und Natrium. Ferner sind als Salzbildner auch Uebergangsmetalle wie beispielsweise

Eisen, Nickel, Kobalt, Kupfer, Zink, Chrom oder Mangan geeignet. Beispiele für zur Salzbildung geeignete Stickstoffbasen sind primäre, sekundäre oder tertiäre, aliphatische und aromatische, gegebenenfalls am Kohlenwasserstoffrest hydroxylierte Amine, wie Methylamin, Ethylamin, Propylamin, Isopropylamin, die vier isomeren Butylamine, Dimethylamin, Diethylamin, Dipropylamin, Diisopropylamin, Di-n-butylamin, Pyrrolidin, Piperidin, Morpholin, Trimethylamin, Triethylamin, Tripropylamin, Chinuclidin, Pyridin, Chinolin, Isochinolin sowie Methanolamin, Ethanolamin, Propanolamin, Dimethanolamin, Diethanolamin oder Triethanolamin. Als organische Stickstoffbasen kommen auch quaternäre Ammoniumbasen in Betracht. Beispiele für quaternäre Ammoniumbasen sind Tetraalkylammoniumkationen, in den die Alkylreste unabhängig voneinander geradkettige oder verzweigte $C_1$-$C_6$-Alkylgruppen sind, wie das Tetramethylammoniumkation, das Tetraethylammoniumkation oder das Trimethylethylammoniumkation, sowie weiterhin das Trimethylbenzylammoniumkation, das Triethylbenzylammoniumkation und das Trimethyl-2-hydroxyethylammoniumkation. Besonders bevorzugt als Salzbildner sind das Ammoniumkation und Trialkylammoniumkationen, in denen die Alkylreste unabhängig voneinander geradkettige oder verzweigte, gegebenenfalls durch eine Hydroxylgruppe substituierte $C_1$-$C_6$-Alkylgruppen, insbesondere $C_1$-$C_2$-Alkylgruppen, sind, wie beispielsweise das Trimethylammoniumkation, das Triethylammoniumkation und das Tri-(2-hydroxyethylen)-ammoniumkation.

Für Z als Carbonsäureamidgruppe kommt ein entsprechender Amidrest in Betracht, welcher unsubstituiert oder am Stickstoffatom mono- oder disubstituiert sein kann oder in welchem das Stickstoffatom Bestandteil eines gegebenenfalls substituierten heterocyclischen Restes ist. Als Substituenten der Amidgruppe sind beispielsweise ein gegebenenfalls substituierter und gegebenenfalls über ein Sauerstoffatom gebundener aliphatischer Rest, ein gegebenenfalls über einen aliphatischen Rest gebundener und gegebenenfalls substituierter cycloaliphatischer, aromatischer oder heterocyclischer Rest oder eine gegebenenfalls mono- oder disubstituierte Aminogruppe zu nennen.

Als Carbonsäureamidrest bevorzugt ist der Rest $-CONR_{14}R_{15}$, worin $R_{14}$ für Wasserstoff, einen gegebenenfalls substituierten Alkyl-, Alkenyl-, Alkinyl- Cycloalkyl-, Phenyl- oder Naphthylrest, einen gegebenenfalls substituierten heterocyclischen Rest oder einen Alkoxyrest, $R_{15}$ für Wasserstoff, Amino, mono- oder disubstituiertes Amino oder einen gegebenenfalls substituierten Alkyl-, Alkenyl-, Cycloalkyl- oder Phenylrest oder $R_{14}$ und $R_{15}$ gemeinsam mit dem Stickstoffatom, an das sie gebunden sind, für einen gegebenenfalls substituierten heterocyclischen Rest stehen.

Als Substituenten der organischen Reste $R_{12}$, $R_{13}$, $R_{14}$ und $R_{15}$ kommen beispielsweise Halogen, Nitro, Cyan, Hydroxy, Alkyl, Halogenalkyl, Alkoxy, welches durch ein oder mehrere Sauerstoffatome unterbrochen sein kann, Alkylthio, Halogenalkoxy, Hydroxyalkoxy, welches durch ein oder mehrere Sauerstoffatome unterbrochen sein kann, Hydroxyalkylthio, Alkoxycarbonyl, Amino, Alkylamino, Dialkylamino, Hydroxyalkylamino, Di-(hydroxyalkyl)-amino, Aminoalkylamino, Cycloalkyl, gegebenenfalls substituiertes Phenyl, gegebenenfalls substituiertes Phenoxy oder ein gegebenenfalls substituierter heterocyclischer Rest in Betracht.

Unter heterocyclischen Resten als Bestandteile des Carbonsäureesterrestes, des Carbonsäurethiolesterrestes und des Carbonsäureamidrestes sind vorzugsweise 5- bis 6-gliedrige, gesättigte oder ungesättigte, gegebenenfalls substituierte monocyclische Heterocyclen mit 1 bis 3 Heteroatomen aus der Gruppe N, O und S zu verstehen, wie beispielsweise Furan, Tetrahydrofuran, Tetrahydropyran, Tetrahydropyrimidin, Pyridin, Piperidin, Morpholin und Imidazol.

Unter Cycloalkylresten als Bestandteile des Carbonsäureesterrestes, des Carbonsäurethiolesterrestes und des Carbonsäureamidrestes sind insbesondere solche mit 3 bis 8, vor allem 3 bis 6 Kohlenstoffatomen, zu verstehen,

Im Substituenten Z als Bestandteil des Carbonsäureesterrestes, des Carbonsäurethiolesterrestes und des Carbonsäureamidrestes vorliegende aliphatische, acyclische Reste können geradkettig oder verzweigt sein und enthalten zweckmässigerweise bis maximal 18 Kohlenstoffatome. Eine geringere Anzahl von Kohlenstoffatomen ist häufig, insbesondere bei zusammengesetzten Substituenten, von Vorteil.

Für Z als cyclisiertes Derivat einer Carbonsäureamidgruppe kommt insbesondere ein gegebenenfalls substituierter Oxazolin-2-yl-Rest, vorzugsweise ein unsubstituierter Oxazolin-2-yl-Rest, in Betracht.

A und Z können zusammen einen gegebenenfalls substituierten Tetrahydrofuran-2-on-Ring bilden, wobei der unsubstituierte Tetrahydrofuran-2-on-Ring bevorzugt ist, insbesondere der unsubstituierte Tetrahydrofuran-2-on-3-yl-Ring.

In den Verbindungen der Formel I bedeutet Halogen Fluor, Chlor, Brom und Jod, insbesondere Chlor, Brom und Jod.

Als Salzbildner kommen organische und anorganische Säuren in Betracht. Beispiele organischer Säuren sind Essigsäure, Trichloressigsäure, Oxalsäure, Benzolsulfonsäure und Methansulfonsäure. Beispiele anorganischer Säuren sind Chlorwasserstoffsäure, Bromwasserstoffsäure, Jodwasserstoffsäure, Schwefelsäure, Phosphorsäure, phosphorige Säure und Salpetersäure.

Als Metallkomplexbildner eignen sich beispielsweise Elemente der 3. und 4. Hauptgruppe, wie Aluminium, Zinn und Blei, sowie der 1. bis 8. Nebengruppe, wie beispielsweise Chrom. Mangan, Eisen, Kobalt, Nickel, Zirkon, Zink, Kupfer, Silber und Quecksilber. Bevorzugt sind die Nebengruppenelemente der 4. Periode.

Wenn in den Verbindungen der Formel I A für $-CH(CH_3)-$ steht oder A und Z zusammen einen Tetrahydrofuran-2-on-Ring bilden, existieren optisch isomere Verbindungen. Im Rahmen der vorliegenden Erfindung sind unter den entsprechenden Verbindungen der Formel I sowohl die optisch reinen Isomere wie auch die Isomerengemische zu verstehen.

Besonders geeignet zur erfindungsgemässen Verwendung sind Verbindungen der Formel I, in denen

a) $R_1$ Wasserstoff oder Halogen bedeutet, wobei als Halogen vorzugsweise Chlor, Jod und Brom in Betracht kommen, $R_2$ für Wasserstoff steht, $R_3$ Wasserstoff, Halogen oder Nitro bedeutet, wobei als Halogen vorzugsweise Chlor und Brom in Betracht kommen, $R_4$ und $R_5$ für Wasserstoff stehen und $R_6$ für Wasserstoff oder $C_1$-$C_4$-Alkyl steht, wobei als Alkyl Methyl bevorzugt ist;

b) A für $-CH_2-$ steht;

c) A für $-CH_2CH_2-$ steht;

d) A für $-CH(CH_3)-$ steht;

e) Z für Amidoxim 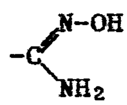 steht;

f) Z für acyliertes Amidoxim 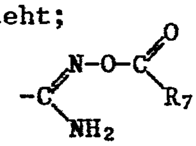 steht, worin $R_7$

unsubstituiertes oder durch Halogen, vorzugsweise Chlor oder Brom, mono- oder disubstituiertes oder durch $C_1$-$C_4$-Alkoxy mono-substituiertes $C_1$-$C_4$-Alkyl bedeutet, oder für Cyclopropyl, $C_2$-$C_3$-Alkenyl, unsubstituiertes oder durch Halogen, vorzugsweise Chlor, monosubstituiertes Phenyl, oder für Benzyl, unsubstituiertes oder durch Halogen, vorzugsweise Brom, substituiertes Furanyl, oder für Tetrahydrofuranyl, Thienyl oder dihalogeniertes Pyrimidin, wie beispielsweise Dichlorpyrimidin, steht;

g) Z acyliertes Amidoxim 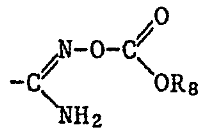 bedeutet, worin

$R_8$ für unsubstituiertes oder durch Halogen, vorzugsweise Brom, monosubstituiertes $C_1$-$C_4$-Alkyl oder für Allyl, Phenyl oder Benzyl steht;

h) Z acyliertes Amidoxim 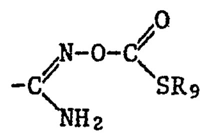 bedeutet, worin

$R_9$ für $C_1$-$C_5$-Alkyl steht;

i) Z acyliertes Amidoxim 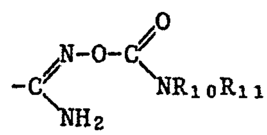 bedeutet, worin

$R_{10}$ für $C_1$-$C_4$-Alkyl oder für unsubstituiertes oder durch Halogen, vorzugsweise Chlor, mono- oder disubstituiertes oder durch Trihalogenmethyl, vorzugsweise Trifluormethyl, monosubstituiertes Phenyl und $R_{11}$ für Wasserstoff oder Methoxy steht;

k) Z für den Carbonsäureesterrest $-COOR_{12}$ steht, worin $R_{12}$ für Wasserstoff, ein Alkalimetallkation, vorzugsweise ein Natrium- oder Kaliumkation, für das Ammoniumkation oder für ein durch $C_1$-$C_4$-Alkyl oder Monohydroxy-$C_1$-$C_4$-Alkyl, beispielsweise 2-Hydroxyethyl, trisubstituiertes Ammoniumkation, für $C_1$-$C_{12}$-Alkyl, insbesondere $C_1$-$C_4$-Alkyl, oder für durch Halogen, vorzugsweise Chlor, oder $C_1$-$C_3$-Alkoxy, Phenoxy, Phenyl oder Tetrahydrofuranyl monosubstituiertes $C_1$-$C_4$-Alkyl, oder für $C_3$-$C_6$-Alkenyl, vorzugsweise Methylallyl, oder $C_3$-$C_4$-Alkinyl, vorzugsweise 2-Propinyl, oder Cyclohexyl oder für unsubstituiertes oder durch Methyl mono- oder disubstituiertes Phenyl steht;

l) Z für den Carbonsäurethiolesterrest $-COSR_{13}$ steht, worin $R_{13}$ $C_5$-$C_{10}$-Alkyl, vorzugsweise n-Octyl, bedeutet;

m) Z für den Carbonsäureamidrest $-CONR_{14}R_{15}$ steht, worin $R_{14}$ Wasserstoff, $C_1$-$C_{12}$-Alkyl, insbesondere $C_1$-$C_4$-Alkyl, oder durch Hydroxy, $C_1$-$C_4$-Alkoxy, Di-($C_1$-$C_4$-alkyl)-amino, (Monohydroxy-$C_1$-$C_4$-alkyl)-amino, Di-(monohydroxy-$C_1$-$C_4$-alkyl)-amino, Phenyl, Tetrahydrofuranyl, Piperidinyl oder Morpholinyl monosubstituiertes $C_1$-$C_4$-Alkyl oder Allyl, Cyclohexyl oder Amino bedeutet und $R_{15}$ Wasserstoff, $C_1$-$C_4$-Alkyl oder Monohydroxy-$C_1$-$C_4$-alkyl bedeutet, oder worin $R_{14}$ und $R_{15}$ gemeinsam mit dem Stickstoffatom, an das sie gebunden sind, den Morpholinring bilden; oder

n) A und Z gemeinsam für Tetrahydrofuran-2-on stehen.

Besonders bevorzugt sind Verbindungen der Formel I, in denen gleichzeitig $R_1$, $R_2$, $R_3$, $R_4$, $R_5$ und $R_6$ eine der vorstehend unter a) angegebenen, A eine der vorstehend unter b) bis d) angegebenen und Z eine der vorstehend unter e) bis m) angegebenen Bedeutungen haben oder A und Z zusammen die unter n) angegebene Bedeutung haben, und insbesondere die folgende Gruppe von Verbindungen der Formel I, in denen $R_1$ Wasserstoff, Chlor, Brom oder Jod, $R_2$ Wasserstoff, $R_3$ Wasserstoff, Chlor, Brom oder Nitro, $R_4$ und $R_5$ Wasserstoff, $R_6$ Wasserstoff oder Methyl, A eine der Gruppen $-CH_2-$, $-CH_2CH_2-$ oder $-CH(CH_3)-$ und Z Cyan,

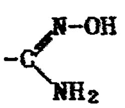 , 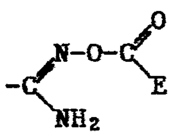 , $-COOR_{12}$, $-COSR_{13}$ oder $-CONR_{14}R_{15}$

bedeuten, worin E $-R_7$, $-OR_8$, $-SR_9$ oder $-NR_{10}R_{11}$ bedeutet und $R_7$ für unsubstituiertes oder durch Chlor oder Brom mono- oder disubstituiertes oder durch $C_1$-$C_4$-Alkoxy monosubstituiertes $C_1$-$C_4$-Alkyl, oder für Cyclopropyl, $C_2$-$C_3$-Alkenyl, unsubstituiertes oder durch Chlor monosubstituiertes Phenyl, oder für Benzyl, unsubstituiertes oder durch Brom monosubstituiertes Furanyl, oder für Tetrahydrofuranyl, Thienyl oder Dichlorpyrimidin steht,

$R_8$ für unsubstituiertes oder durch Brom monosubstituiertes $C_1$-$C_4$-Alkyl, oder für Allyl, Phenyl oder Benzyl steht,

$R_9$ für $C_1$-$C_5$-Alkyl steht,

$R_{10}$ für $C_1$-$C_4$-Alkyl oder unsubstituiertes oder durch Chlor mono- oder disubstituiertes oder durch Trifluormethyl monosubstituiertes Phenyl steht,

$R_{11}$ für Wasserstoff oder Methoxy steht,

$R_{12}$ Wasserstoff, das Natriumkation, das Kaliumkation, das Ammoniumkation, durch $C_1$-$C_4$-Alkyl oder 2-Hydroxyethyl trisubstituiertes Ammoniumkation, $C_1$-$C_4$-Alkyl, durch Chlor, $C_1$-$C_3$-Alkoxy, Phenoxy, Phenyl oder Tetrahydrofuranyl monosubstituiertes $C_1$-$C_4$-Alkyl, oder Methylallyl, 2-Propinyl, Cyclohexyl oder unsubstituiertes oder durch Methyl mono- oder disubstiuiertes Phenyl bedeutet,

$R_{13}$ n-Octyl bedeutet,

$R_{14}$ Wasserstoff, $C_1$-$C_4$-Alkyl, durch Hydroxy, $C_1$-$C_4$-Alkoxy, Di-($C_1$-$C_4$-alkyl)-amino, (Monohydroxy-$C_1$-$C_4$-alkyl)-amino, Di-(monohydroxy-$C_1$-$C_4$-alkyl)-amino, Phenyl, Tetrahydrofuranyl, Piperidinyl oder Morpholinyl monosubstituiertes $C_1$-$C_4$-Alkyl, oder Allyl, Cyclohexyl oder Amino bedeutet, und

$R_{15}$ Wasserstoff, $C_1$-$C_4$-Alkyl oder Monohydroxy-$C_1$-$C_4$-Alkyl bedeutet, oder

$R_{14}$ und $R_{15}$ gemeinsam mit dem Stickstoffatom, an welches sie gebunden sind, den Morpholinring bilden, oder worin

A und Z gemeinsam für Tetrahydrofuran-2-on stehen.

Von besonderem Interesse sind Verbindungen der Formel I, in denen $R_1$, $R_2$, $R_4$, $R_5$ und $R_6$ Wasserstoff, $R_3$ Wasserstoff oder Chlor, A die Gruppe $-CH_2-$ oder $-CH(CH_3)-$ und Z eine Carbonsäureestergruppe $-COOR_{12}$, worin $R_{12}$ für $C_1$-$C_{12}$-Alkyl oder durch Phenyl monosubstituiertes $C_1$-$C_4$-Alkyl oder für $C_3$-$C_6$-Alkenyl steht, bedeuten.

Beispiele für erfindungsgemäss zu verwendende Verbindungen zeigt die nachfolgende Tabelle 1.

Besonders erwähnenswerte Einzelverbindungen sind O-(Methoxycarbonyl)-2-(8-chinolinoxy)-acetamidoxim und 2-(5-Chlor-8-chinolinoxy)-essigsäure-methylallylester.

Besonders erwähnenswert ist die Verwendung von O-Methoxycarbonyl-2-(8-chinolinoxy)-acetamidoxim oder 2-(5-Chlor-8-chinolinoxy)-essigsäuremethylallylester zum Schützen von Kulturpflanzen, insbesondere Getreide, gegen die schädigende Wirkung von 2-[4-(6-Chlorbenzoxazol-2-yl-oxy)-phenoxy]-propionsäure-ethylester, 2-[4-(6-Chlorbenzthiazol-2-yl-oxy)-phenoxy]-propionsäure-ethylester oder 2-[4-(6-Chlorchinoxalin-2-yl-oxy)-phenoxy]-propionsäure-ethylester.

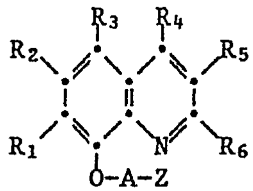

(I),

Tabelle 1

| Nr. | $R_1$ | $R_2$ | $R_3$ | $R_4$ | $R_5$ | $R_6$ | A | Z | physikal. Konstante |
|---|---|---|---|---|---|---|---|---|---|
| 1 | H | H | H | H | H | H | $-CH_2-$ | -CN | Smp. 118-119°C |
| 2 | H | H | H | H | H | H | $-CH_2-$ | 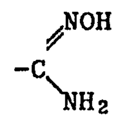 | Smp. 201-204°C (Z) |
| 3 | H | H | H | H | H | $CH_3$ | $-CH_2-$ | -CN | Smp. 114-116°C |
| 4 | H | H | H | H | H | $CH_3$ | $-CH_2-$ | 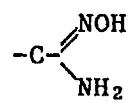 | Smp. 209-210°C (Z) |
| 5 | H | H | Cl | H | H | H | $-CH_2-$ | 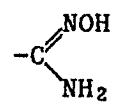 | Smp. 203-205°C (Z) |
| 6 | H | H | H | H | H | H | $-CH_2-$ | 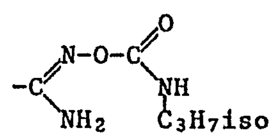 | Smp. 136-138°C |
| 7 | H | H | Cl | H | H | H | $-CH_2-$ | -CN | Smp. 159-160°C |

-13-

0159290

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 8 | H | H | H | H | H | H | $-CH_2-$ | 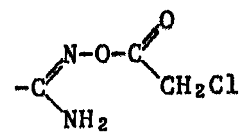 | Smp. 129-130°C |
| 9 | Br | H | Cl | H | H | H | $-CH_2-$ | 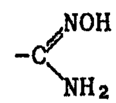 | Smp. 197-198°C (Z) |
| 10 | Br | H | Cl | H | H | H | $-CH_2-$ | -CN | Smp. 150-151°C |
| 11 | H | H | H | H | H | H | $-CH_2-$ | 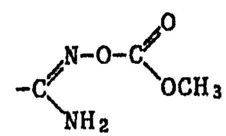 | Smp. 143-145°C |
| 12 | J | H | Cl | H | H | H | $-CH_2-$ | 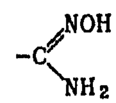 | Smp. 195-196°C (Z) |
| 13 | J | H | Cl | H | H | H | $-CH_2-$ | -CN | Smp. 141-143°C |
| 14 | Br | H | Cl | H | H | H | $-CH_2-$ | 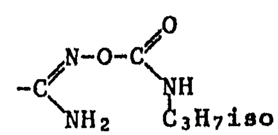 | Smp. 162-165°C |
| 15 | Cl | H | Cl | H | H | $CH_3$ | $-CH_2-$ | 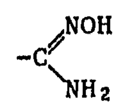 | Smp. 205-207°C (Z) |

0159290

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 16 | Cl | H | Cl | H | H | H | $-CH_2-$ | -CN | Smp. 150-152°C |
| 17 | J | H | Cl | H | H | H | $-CH_2-$ | 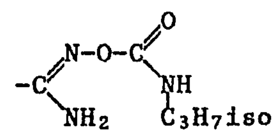 | Smp. 163-167°C |
| 18 | Cl | H | Cl | H | H | $CH_3$ | $-CH_2-$ | -CN | Smp. 157-158°C |
| 19 | Cl | H | Cl | H | H | $CH_3$ | $-CH_2-$ | 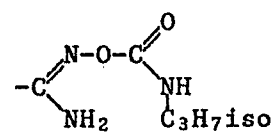 | Smp. 149-152°C |
| 20 | H | H | H | H | H | H | 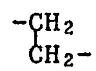 | -CN | Smp. 108-112°C |
| 21 | H | H | H | H | H | H | 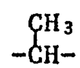 | -CN | Smp. 121-124°C |
| 22 | H | H | H | H | H | H | 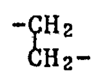 | 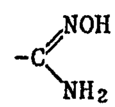 | Smp. 186-189°C |
| 23 | H | H | Cl | H | H | H | 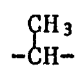 | -CN | Smp. 143-145° |
| 24 | H | H | H | H | H | H | 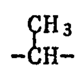 | 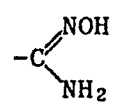 | Smp. 191-194°C (Z) |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 25 | H | H | Cl | H | H | H | 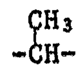 | 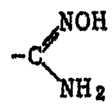 | Smp. 186-189°C (Z) |
| 26 | H | H | $NO_2$ | H | H | H | 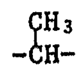 | -CN | Smp. 154-156°C |
| 27 | Cl | H | $NO_2$ | H | H | H | $-CH_2-$ | 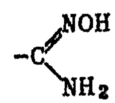 | Smp. 214-216°C (Z) |
| 28 | Cl | H | $NO_2$ | H | H | H | $-CH_2-$ | -CN | Smp. 166-169°C |
| 29 | H | H | H | H | H | H | $-CH_2-$ | 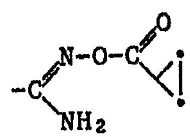 | Smp. 165-166°C |
| 30 | H | H | H | H | H | H | $-CH_2-$ | 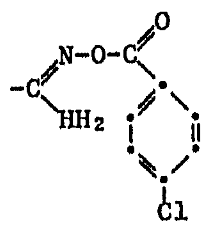 | Smp. 139-141°C |
| 31 | H | H | Cl | H | H | H | $-CH_2-$ | 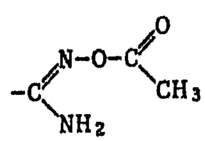 | Smp. 141-143°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 32 | H | H | $NO_2$ | H | H | H | $-CH_2-$ | -CN | Smp. 162-164°C |
| 33 | H | H | $NO_2$ | H | H | H | $-CH_2-$ | 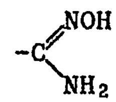 | Smp. 212-215°C (Z) |
| 34 | H | H | Cl | H | H | H | $-CH_2-$ | 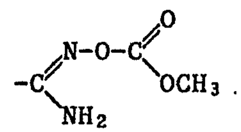 | Smp. 148-149°C |
| 35 | H | H | Cl | H | H | H | $-CH_2-$ | 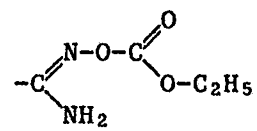 | Smp. 139-140°C |
| 36 | H | H | H | H | H | H | $-CH_2-$ | 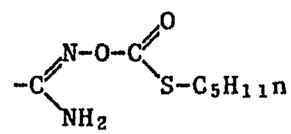 | Smp. 111-114°C |
| 37 | H | H | H | H | H | H | $-CH_2-$ | 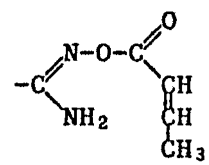 | Smp. 158-162°C |
| 38 | H | H | H | H | H | H | $-CH_2-$ | 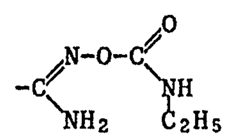 | Smp. 123-125°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 39 | H | H | H | H | H | H | $-CH_2-$ | 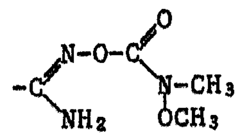 | Smp. 138-139°C |
| 40 | H | H | H | H | H | H | $-CH_2-$ | 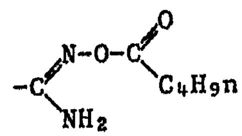 | Smp. 120-122°C |
| 41 | H | H | Cl | H | H | H | $-CH_2-$ | 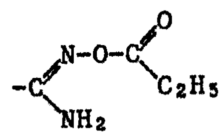 | Smp. 157-158°C (Z) |
| 42 | H | H | H | H | H | H | $-CH_2-$ | 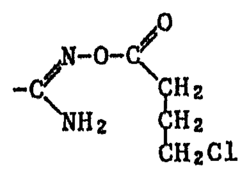 | Smp. 144-146°C |
| 43 | H | H | H | H | H | H | $-CH_2-$ | 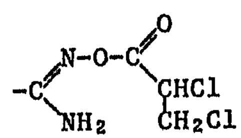 | Smp. 112-114°C |
| 44 | H | H | Cl | H | H | H | $-CH_2-$ | 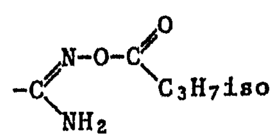 | Smp. 173-174°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 45 | H | H | H | H | H | H | $-CH_2-$ | 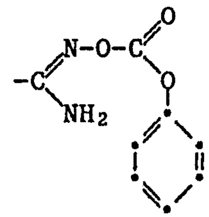 | Smp. 155-156°C |
| 46 | H | H | H | H | H | H | $-CH_2-$ | 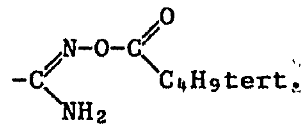 | Smp. 107-110,5°C |
| 47 | H | H | H | H | H | H | $-CH_2-$ | 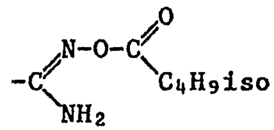 | Smp. 124-126°C |
| 48 | H | H | H | H | H | H | $-CH_2-$ | 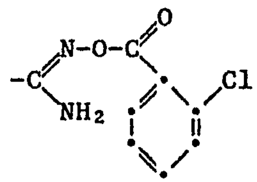 | Smp. 131-132°C |
| 49 | H | H | H | H | H | H | $-CH_2-$ | 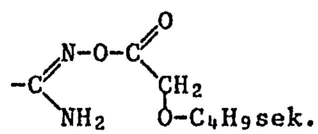 | Smp. 84-86°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 50 | H | H | H | H | H | H | $-CH_2-$ | 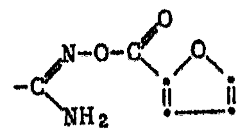 | Smp. 168-169°C |
| 51 | H | H | H | H | H | H | $-CH_2-$ | 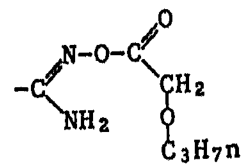 | Smp. 100-103°C |
| 52 | H | H | Cl | H | H | H | $-CH_2-$ | 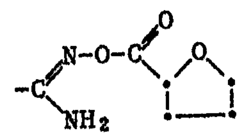 | Smp. 156-157°C (Z) |
| 53 | H | H | H | H | H | H | $-CH_2-$ | 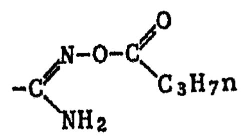 | Smp. 82-85°C |
| 54 | H | H | H | H | H | H | $-CH_2-$ | 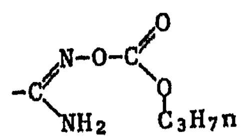 | Smp. 144-147°C |
| 55 | H | H | H | H | H | H | $-CH_2-$ | 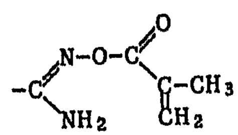 | Smp. 128-130°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 56 | H | H | H | H | H | H | $-CH_2-$ | 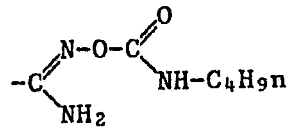 | Smp. 90-92°C |
| 57 | H | H | H | H | H | H | $-CH_2-$ | 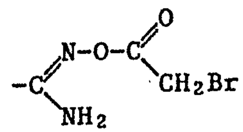 | Smp. 132-134°C |
| 58 | H | H | H | H | H | H | $-CH_2-$ | 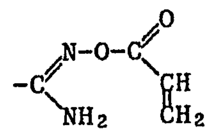 | Smp. 138-140°C |
| 59 | H | H | H | H | H | H | $-CH_2-$ | 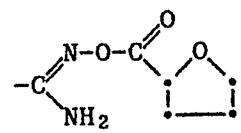 | Smp. 129-131°C |
| 60 | H | H | H | H | H | H | $-CH_2-$ | 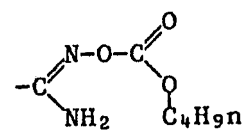 | Smp. 121-123°C |
| 61 | H | H | H | H | H | H | $-CH_2-$ | 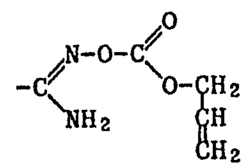 | Smp. 123-125°C |

<u>Tabelle 1</u> (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 62 | H | H | H | H | H | H | $-CH_2-$ | 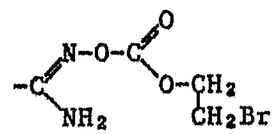 | Smp. 127-128°C (Z) |
| 63 | H | H | Cl | H | H | H | $-CH_2-$ | 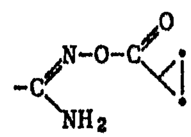 | Smp. 173-175°C |
| 64 | H | H | H | H | H | H | $-CH_2-$ | 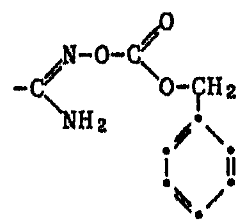 | Smp. 135-137°C |
| 65 | H | H | Cl | H | H | H | $-CH_2-$ | 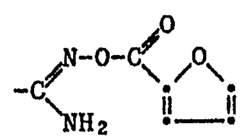 | Smp. 191-192°C (Z) |
| 66 | H | H | H | H | H | H | $-CH_2-$ | 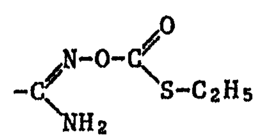 | Smp. 120-121°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 67 | H | H | H | H | H | H | $-CH_2-$ | 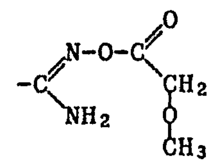 | Smp. 118-120°C |
| 68 | H | H | Cl | H | H | H | $-CH_2-$ | 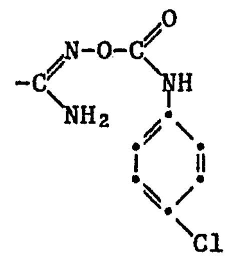 | Smp. 191-192°C (Z) |
| 69 | H | H | H | H | H | H | $-CH_2-$ | 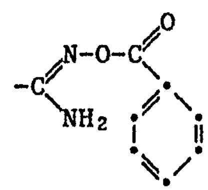 | Smp. 158-159°C |
| 70 | H | H | H | H | H | H | $-CH_2-$ | 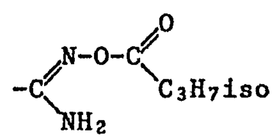 | Smp. 115-117,5°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 71 | H | H | H | H | H | H | $-CH_2-$ | 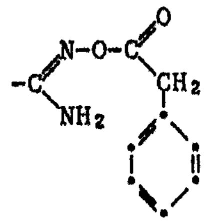 | Smp. 140-142°C |
| 72 | H | H | H | H | H | H | $-CH_2-$ | 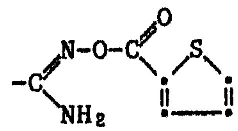 | Smp. 164-165°C |
| 73 | H | H | H | H | H | H | $-CH_2-$ | 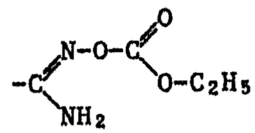 | Smp. 129-132°C |
| 74 | H | H | H | H | H | H | $-CH_2-$ | 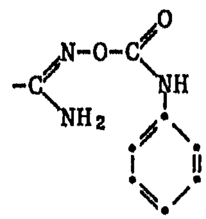 | Smp. 155-157,5°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 75 | H | H | H | H | H | H | $-CH_2-$ | 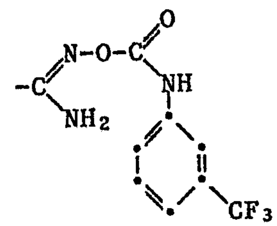 | Smp. 158-160°C |
| 76 | H | H | Cl | H | H | H | $-CH_2-$ | 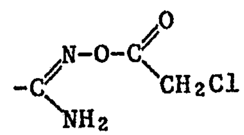 | Smp. 155-158°C (Z) |
| 77 | H | H | H | H | H | H | $-CH_2-$ | 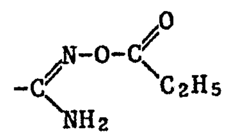 | Smp. 144-146°C |
| 78 | H | H | H | H | H | H | $-CH_2-$ | 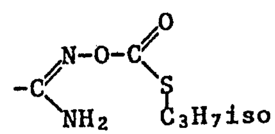 | Smp. 123-124°C |

-25-

0159290

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 79 | H | H | H | H | H | H | $-CH_2-$ | 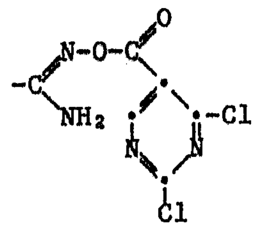 | Smp. 173-176°C (Z) |
| 80 | H | H | H | H | H | H | $-CH_2-$ | 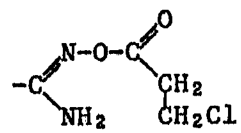 | Smp. 134-136°C (Z) |
| 81 | H | H | H | H | H | H | $-CH_2-$ | 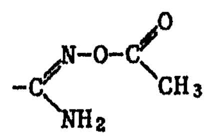 | Smp. 100-102°C |
| 82 | H | H | H | H | H | H | $-CH_2-$ | 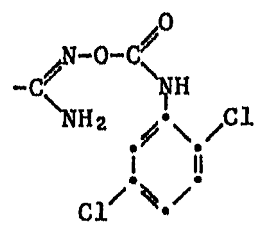 | Smp. 197-199°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 83 | H | H | H | H | H | H | $-CH_2-$ | 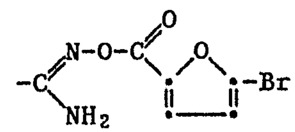 | Smp. 170-171°C |
| 84 | Cl | H | Cl | H | H | H | $-\underset{CH_3}{CH}-$ | $-COOCH_3$ | Smp. 65-66°C |
| 85 | H | H | H | H | H | H | $-\underset{CH_3}{CH}-$ | $-COOCH_3$ | Smp. 70-72°C |
| 86 | H | H | H | H | H | H | $-CH_2-$ | $-COOH \cdot H_2O$ | Smp. 184-185°C |
| 87 | H | H | H | H | H | H | $-CH_2-$ | $-COOCH_2CH_2OCH_3$ | Smp. 80-82°C |
| 88 | H | H | H | H | H | H | $-CH_2-$ | $-COOCH_3$ | Smp. 46,5-67,0°C |
| 89 | H | H | H | H | H | H | $-CH_2-$ | $-COOC_2H_5 \cdot H_2O$ | Smp. 56-59°C |
| 90 | H | H | H | H | H | H | $-\underset{CH_3}{CH}-$ | $-CONH(CH_2)_3OC_2H_5$ | Smp. 54-56°C |
| 91 | H | H | H | H | H | H | $-\underset{CH_3}{CH}-$ | $-CONHC_2H_5$ | Smp. 86-88°C |
| 92 | H | H | H | H | H | H | $-CH_2-$ | $-COOC_3H_7n$ | Smp. 28-31°C |
| 93 | H | H | H | H | H | H | $-CH_2-$ | $-COOC_3H_7iso$ | $n_D^{23} = 1,5696$ |
| 94 | H | H | H | H | H | H | $-CH_2-$ | $-CONHCH_3 \cdot H_2O$ | Smp. 74-81°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 95 | H | H | H | H | H | H | $-CH_2-$ | $CH_3$<br>$-CON$<br>$CH_3$ | Smp. 142-145°C |
| 96 | H | H | H | H | H | H | $-CH_2-$ | $-CONHC_2H_5$ | $n_D^{22,5} = 1,6002$ |
| 97 | H | H | H | H | H | H | $-CH-$<br>$CH_3$ | $-CONH(CH_2)_3OH$ | Smp. 120-122°C |
| 98 | H | H | H | H | H | H | $-CH_2-$ | $-COOCH_2CH_2OC_2H_5$ | $n_D^{24} = 1,5673$ |
| 99 | H | H | H | H | H | H | $-CH-$<br>$CH_3$ | $-CONHCH_2-$ 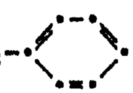 | Smp. 88-90°C |
| 100 | H | H | H | H | H | H | $-CH_2-$ | $-CONH(CH_2)_3CH_3$ | Smp. 66-68°C |
| 101 | H | H | H | H | H | H | $-CH-$<br>$CH_3$ | $CH_3$<br>$-CON$<br>$CH_2CH_2OH$ | $n_D^{22} = 1,6054$ |
| 102 | H | H | H | H | H | H | $-CH_2-$ | $CH_3$<br>$-CON$<br>$CH_2CH_2OH$ | Smp. 146-149°C |
| 103 | H | H | H | H | H | H | $-CH_2-$ | $-COOCH_2-$ 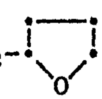 | zähe Masse |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 104 | H | H | H | H | H | H | 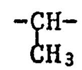 | $-CONH(CH_2)_3CH_3 \cdot H_2O$ | Smp. 73-76°C |
| 105 | H | H | H | H | H | H | 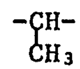 | 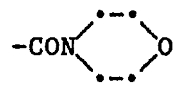 | Smp. 120-121°C |
| 106 | H | H | H | H | H | H | 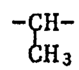 | 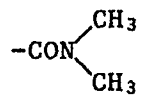 | Smp. 105-111°C |
| 107 | H | H | Cl | H | H | H | $-CH_2-$ | $-COOH$ | Smp. 232-233°C |
| 108 | H | H | Cl | H | H | H | $-CH_2-$ | $-COOCH_2CH_2OCH_3$ | Smp. 97-98°C |
| 109 | H | H | Cl | H | H | H | $-CH_2-$ | $-COOCH_3$ | Smp. 104-105,5°C |
| 110 | H | H | Cl | H | H | H | $-CH_2-$ | $-COOC_2H_5$ | Smp. 116-117°C |
| 111 | H | H | Cl | H | H | H | $-CH_2-$ | $-COOC_3H_7n$ | Smp. 108-109°C |
| 112 | H | H | Cl | H | H | H | $-CH_2-$ | 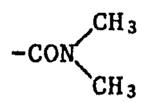 | Smp. 135-136°C |
| 113 | H | H | H | H | H | $CH_3$ | $-CH_2-$ | $-COOCH_3$ | Smp. 58-66°C |
| 114 | H | H | H | H | H | $CH_3$ | $-CH_2-$ | $-COOC_2H_5$ | $n_D^{22,5}$ = 1,5762 |
| 115 | H | H | Cl | H | H | H | $-CH_2-$ | $-COOC_4H_9tert.$ | Smp. 63-69°C |
| 116 | H | H | H | H | H | H | $-CH_2-$ | $-COOC_4H_9tert.$ | Smp. 68-70°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 117 | H | H | Cl | H | H | H | $-CH_2-$ | $-COOCH_2-C\equiv CH$ | Smp. 115-116°C |
| 118 | H | H | Cl | H | H | H | $-CH_2-$ | $-COOC_3H_7$iso | Smp. 147-148°C |
| 119 | H | H | Cl | H | H | H | $-CH_2-$ | $-COOCH_2CH_2OC_2H_5$ | Smp. 102-104°C |
| 120 | H | H | Cl | H | H | H | $-CH_2-$ | $-COOCH_2-$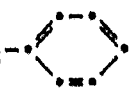 | Smp. 110-112°C |
| 121 | H | H | Cl | H | H | H | $-CH_2-$ | $-COOCH_2-CH=CH_2$ | Smp. 98-99°C |
| 122 | H | H | Cl | H | H | H | $-CH_2-$ | $-COO(CH_2)_{11}CH_3$ | Smp. 76-77°C |
| 123 | H | H | Cl | H | H | H | $-CH_2-$ | $-COOC_4H_9$sek. | Smp. 110-111°C |
| 124 | H | H | H | H | H | H | $-CH_2-$ | $-COO(CH_2)_7CH_3$ | $n_D^{24} = 1{,}5419$ |
| 125 | H | H | Cl | H | H | H | $-CH_2-$ | $-COOC_4H_9$n | Smp. 90,5-92°C |
| 126 | H | H | H | H | H | H | $-CH_2-$ | $-COO(CH_2)_{11}CH_3$ | $n_D^{23} = 1{,}5232$ |
| 127 | H | H | H | H | H | H | $-CH_2-$ | $-COOCH_2-CH=CH_2$ | $n_D^{23} = 1{,}5885$ |
| 128 | H | H | Cl | H | H | H | $-CH_2-$ | $-COO(CH_2)_7CH_3$ | Smp. 87-88°C |
| 129 | H | H | H | H | H | H | $-CH_2-$ | $-COOC_4H_9$n | $n_D^{22} = 1{,}5642$ |
| 130 | H | H | H | H | H | H | $-CH_2-$ | $-COOC_4H_9$sek. | Oel (rot) |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 131 | H | H | Cl | H | H | H | $-CH_2-$ | $-COOCH_2CH_2Cl$ | Smp. 125-126°C |
| 132 | H | H | H | H | H | H | $-CH_2-$ | $-COOCH_2-$ 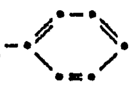 | $n_D^{23,5}$ = 1,6099 |
| 133 | H | H | Cl | H | H | H | $-CH_2-$ | $-COOCH_2-$ 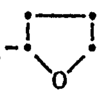 | Smp. 101-103°C |
| 134 | H | H | Cl | H | H | H | $-CH_2-$ | $-COS(CH_2)_7CH_3$ | Smp. 53-54°C |
| 135 | H | H | H | H | H | H | $-CH_2-$ | $-COOCH_2CH_2Cl$ | Smp. 109-110°C |
| 136 | J | H | Cl | H | H | H | $-CH_2-$ | $-COOC_4H_9$tert. | Smp. 81-97°C |
| 137 | J | H | Cl | H | H | H | $-CH_2-$ | $-COOC_2H_5$ | Smp. 92-94°C |
| 138 | J | H | Cl | H | H | H | $-CH_2-$ | $-COO(CH_2)_{11}CH_3$ | Smp. 51-53°C |
| 139 | J | H | Cl | H | H | H | $-CH_2-$ | $-COOCH_3$ | Smp. 121-126°C |
| 140 | J | H | Cl | H | H | H | $-CH_2-$ | $-COOCH_2CH_2Cl$ | Smp. 44-45°C |
| 141 | J | H | Cl | H | H | H | $-CH_2-$ | $-COOCH_2-$ 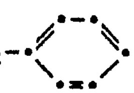 | Smp. 112-113°C |
| 142 | J | H | Cl | H | H | H | $-CH_2-$ | $-COOC_3H_7n$ | Smp. 71-73°C |
| 143 | H | H | H | H | H | H | $-CH_2-$ | $-COOC_4H_9iso$ | $n_D^{22}$ = 1,5632 |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 144 | H | H | H | H | H | H | $-CH_2-$ | $-COOCHCH_2CH_2CH_3$<br>$CH_3$ | $n_D^{22}$ = 1,5391 |
| 145 | H | H | H | H | H | H | $-CH_2-$ | $-COOCH(CH_2)_5CH_3$<br>$CH_3$ | $n_D^{22}$ = 1,5342 |
| 146 | H | H | H | H | H | H | $-CH_2-$ | $-CONH(CH_2)_{11}CH_3$ | Smp. 56-61°C |
| 147 | H | H | H | H | H | H | $-CH_2-$ | $-CONHCH_2CH_2-$ 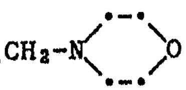 | Smp. 94-99°C |
| 148 | H | H | H | H | H | H | $-CH_2-$ | $-CONHCH_2CH_2CH_2OH$ | Smp. 138-139°C |
| 149 | H | H | H | H | H | H | $-CH_2-$ | $-CONH-$ 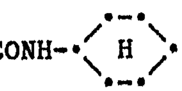 | Smp. 104-106°C |
| 150 | H | H | H | H | H | H | $-CH_2-$ | $-CO$ 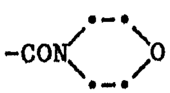 | Smp. 99-103°C |
| 151 | H | H | H | H | H | H | $-CH_2-$ | 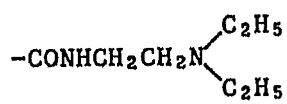 | $n_D^{23}$ = 1,5686 |
| 152 | H | H | H | H | H | H | $-CH_2-$ | 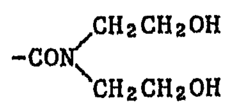 | Smp. 144-146°C |



0159290

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 153 | H | H | H | H | H | H | $-CH_2-$ | 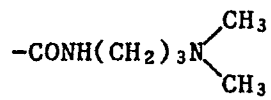 | $n_D^{23}$ = 1,5766 |
| 154 | H | H | H | H | H | H | $-CH_2-$ | 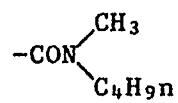 | $n_D^{22}$ = 1,5840 |
| 155 | H | H | H | H | H | H | $-CH_2-$ | 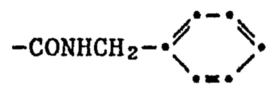 • $H_2O$ | Smp. 70,5-73,5°C |
| 156 | H | H | H | H | H | H | $-CH_2-$ | 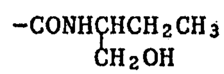 | Smp. 150-151°C |
| 157 | H | H | H | H | H | H | $-CH_2-$ | 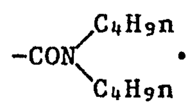 • $2H_2O$ | Smp. 105-106°C |
| 158 | H | H | H | H | H | H | $-CH_2-$ | 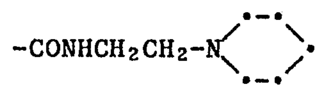 | $n_D^{26}$ = 1.5821 |
| 159 | H | H | H | H | H | H | $-CH_2-$ | 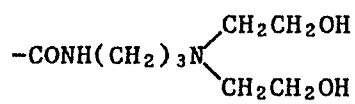 | Smp. 109-110°C |
| 160 | H | H | H | H | H | H | $-CH_2-$ | $-CONHCH_2-CH=CH_2$ • $H_2O$ | Smp. 71-75°C |



0159290

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 161 | H | H | H | H | H | H | $-CH_2-$ | $-CONHCH_2-$ 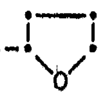 $\cdot H_2O$ | Smp. 57-58°C |
| 162 | H | H | H | H | H | H | $-CH_2-$ | $-CONH(CH_2)_3OC_2H_5$ | Smp. 51-61°C |
| 163 | H | H | H | H | H | H | $-CH_2-$ | $-CONHCH_2CH_2NHCH_2CH_2OH$ | Smp. 70-91°C |
| 164 | H | H | Cl | H | H | H | $-CH_2-$ | $CONH(CH_2)_3OC_2H_5$ | Smp. 85-88°C |
| 165 | H | H | Cl | H | H | H | $-CH_2-$ | 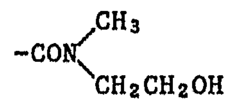 | Smp. 187-189°C |
| 166 | H | H | Cl | H | H | H | $-CH_2-$ | 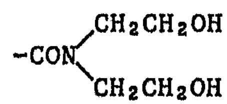 | Smp. 177-179°C |
| 167 | H | H | Cl | H | H | H | $-CH_2-$ | 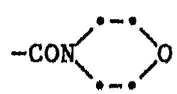 | Smp. 148-150°C |
| 168 | H | H | Cl | H | H | H | $-CH_2-$ | $-CONHCH_2CH_2CH_2OH$ | Smp. 157-160°C |
| 169 | H | H | Cl | H | H | H | $-CH_2-$ | $-CONHC_4H_9n \cdot H_2O$ | Smp. 87-90°C |
| 170 | H | H | Cl | H | H | H | $-CH_2-$ | $-CONHC_2H_5$ | Smp. 94-98°C |
| 171 | H | H | Cl | H | H | H | $-CH_2-$ | $-CONHCH_2-$ 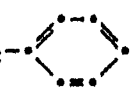 $\cdot \frac{1}{2} H_2O$ | Smp. 146-149°C |

-34-

0159290

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 172 | H | H | H | H | H | $CH_3$ | $-CH_2-$ | $-CONH_2$ | Smp. 193-196°C |
| 173 | H | H | H | H | H | H | $-CH_2-$ | $-CONHNH_2 \cdot H_2O$ | Smp. 121-124°C |
| 174 | H | H | H | H | H | H | $-CH_2-$ | $-COONa \cdot H_2O$ | Smp. 140-142°C |
| 175 | H | H | H | H | H | H | $-CH_2-$ | $-COOK \cdot H_2O$ | Smp. > 200°C |
| 176 | H | H | H | H | H | H | $-CH_2-$ | $-COO^{\ominus}\ \overset{\oplus}{H}N(CH_3)_3$ | Smp. 176-178°C |
| 177 | H | H | H | H | H | H | $-CH_2-$ | $-COO^{\ominus}\ \overset{\oplus}{H}N(CH_2CH_2OH)_3$ | Smp. 97-98°C |
| 178 | H | H | Cl | H | H | H | $-CH_2-$ | $-COOK \cdot H_2O$ | Smp. > 260°C |
| 179 | H | H | Cl | H | H | H | $-CH_2-$ | $-COONa \cdot H_2O$ | Smp. > 260°C |
| 180 | H | H | H | H | H | H | $-CH_2-$ | $-COO^{\ominus}\ \overset{\oplus}{H}N(C_2H_5)_3$ | Smp. 255-257°C (Z) |
| 181 | H | H | Cl | H | H | H | $-CH_2-$ | $-COO^{\ominus}\ {}^{\oplus}NH_4$ | Smp. 227-228°C (Z) |
| 182 | H | H | Cl | H | H | H | $-CH_2-$ | $-COO^{\ominus}\ \overset{\oplus}{H}N(CH_2CH_2OH)_3$ | Smp. 132-156°C (Z) |
| 183 | H | H | Cl | H | H | H | $-\underset{CH_3}{CH}-$ | -COO- 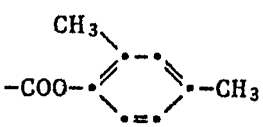 | Smp. 120-122°C |
| 184 | H | H | Cl | H | H | H | $-CH_2-$ | $-COO\overset{CH_3}{CH}(CH_2)_5CH_3$ | Smp. 65-67°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 185 | H | H | Cl | H | H | H | $-CH_2-$ | $-COOCH_2CH=CH-CH_3$ | Smp. 100-102°C |
| 186 | H | H | Cl | H | H | H | $-CH_2-$ | $-COOCH_2-\overset{CH_3}{C}=CH_2$ | Smp. 94-95°C |
| 187 | H | H | Cl | H | H | H | $-CH_2-$ | $-COOCH_2CH_2OC_3H_7iso$ | Smp. 70-72°C |
| 188 | H | H | Cl | H | H | H | $-CH_2-$ | $-COOCH_2CH_2-O-$ 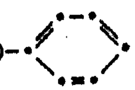 | Smp. 79-80,5°C |
| 189 | Br | H | Br | H | H | H | $-CH_2-$ | $-COOCH_3$ | Smp. 143-145°C |
| 190 | Br | H | Cl | H | H | H | $-CH_2-$ | $-COOC_3H_7iso$ | Smp. 71-73°C |
| 191 | Br | H | Br | H | H | H | $-CH_2-$ | $-COOC_3H_7iso$ | Smp. 47-51°C |
| 192 | Cl | H | Cl | H | H | H | $-CH_2-$ | $-COOC_4H_9n$ | Smp. 42-43,5°C |
| 193 | Br | H | Cl | H | H | H | $-CH_2-$ | $-COOC_4H_9n$ | Smp. ca. 28°C |
| 194 | Cl | H | Cl | H | H | H | $-CH_2-$ | $-COO(CH_2)_7CH_3$ | Smp. ca. 30°C |
| 195 | Br | H | Cl | H | H | H | $-CH_2-$ | $-COO(CH_2)_7CH_3$ | Smp. 41-42°C |
| 196 | Br | H | Cl | H | H | H | $-CH_2-$ | $-COO\overset{CH_3}{C}H(CH_2)_5CH_3$ | Smp. 46-48°C |
| 197 | Cl | H | Cl | H | H | H | $-CH_2-$ | $-COO(CH_2)_{11}CH_3$ | Smp. 49-50°C |
| 198 | Br | H | Cl | H | H | H | $-CH_2-$ | $-COO(CH_2)_{11}CH_3$ | Smp. 50-52°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 199 | Cl | H | Cl | H | H | H | $-CH_2-$ | $-COOCH_2-$ 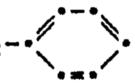 | Smp. 79-80°C |
| 200 | Br | H | Cl | H | H | H | $-CH_2-$ | $-COOCH_2-$ 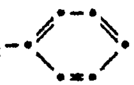 | Smp. 100-102°C |
| 201 | Br | H | Br | H | H | H | $-CH_2-$ | $-COOCH_2-$ 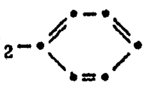 | Smp. 101-104°C |
| 202 | Br | H | Cl | H | H | H | $-CH_2-$ | $-COOCH_2CH_2OCH_3$ | Smp. 68-70°C |
| 203 | Cl | H | Cl | H | H | H | $-CH_2-$ | $-COOCH_2CH_2OC_2H_5$ | Smp. 81-82°C |
| 204 | Br | H | Cl | H | H | H | $-CH_2-$ | $-COOCH_2CH_2OC_2H_5$ | Smp. 71-72°C |
| 205 | Br | H | Cl | H | H | H | $-CH_2-$ | $-COOCH_2CH_2OC_3H_7$iso | $n_D^{25}$ = 1,5763 |
| 206 | Br | H | Cl | H | H | H | $-CH_2-$ | $-COOCH_2CH_2O-$ 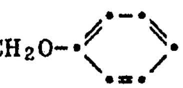 | Smp. 80-82°C |
| 207 | Cl | H | Cl | H | H | H | $-CH_2-$ | $-COOCH_2-$ 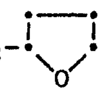 | Smp. 77-78°C |
| 208 | Br | H | Cl | H | H | H | $-CH_2-$ | $-COOCH_2-$ 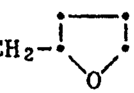 | Smp. 79-80°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 209 | Cl | H | Cl | H | H | H | $-CH_2-$ | $-COOCH_2CH{=}CH_2$ | Smp. 72-73°C |
| 210 | Br | H | Cl | H | H | H | $-CH_2-$ | $-COOCH_2CH{=}CH_2$ | Smp. 66-68,5°C |
| 211 | Br | H | Br | H | H | H | $-CH_2-$ | $-COOCH_2CH{=}CH_2$ | Smp. 78-79°C |
| 212 | Br | H | Cl | H | H | H | $-CH_2-$ | $-COOCH_2CH{=}CH-CH_3$ | Smp. 60-64°C |
| 213 | Cl | H | Cl | H | H | H | $-CH_2-$ | $-COOCH_2-C(CH_3){=}CH_2$ | Smp. 62-65°C |
| 214 | Br | H | Cl | H | H | H | $-CH_2-$ | $-COOCH_2-C(CH_3){=}CH_2$ | Smp. 62-64°C |
| 215 | Br | H | Cl | H | H | H | $-CH_2-$ | -COO- 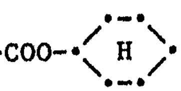 | Smp. 52-54°C |
| 216 | H | H | Cl | H | H | H | $-CH(CH_3)-$ | $-COOC_3H_7$iso | $n_D^{24}$ = 1,5642 |
| 217 | H | H | Cl | H | H | H | $-CH(CH_3)-$ | $-COO(CH_2)_7CH_3$ | $n_D^{23}$ = 1,5356 |
| 218 | H | H | Cl | H | H | H | $-CH(CH_3)-$ | $-COOCH(CH_3)(CH_2)_5CH_3$ | $n_D^{25}$ = 1,5370 |
| 219 | H | H | Cl | H | H | H | $-CH(CH_3)-$ | $-COO(CH_2)_{11}CH_3$ | Smp. 54-55°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 220 | H | H | Cl | H | H | H | $-\underset{CH_3}{CH}-$ | $-COOCH_2-$ 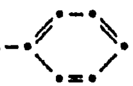 | Smp. 57-59°C |
| 221 | H | H | Cl | H | H | H | $-\underset{CH_3}{CH}-$ | $-COOCH_2CH_2OC_3H_7iso$ | $n_D^{32}$ = 1,5403 |
| 222 | H | H | Cl | H | H | H | $-\underset{CH_3}{CH}-$ | $-COOCH_2CH_2O-$ 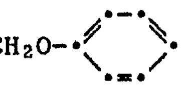 | $n_D^{29}$ = 1,5962 |
| 223 | H | H | Cl | H | H | H | $-\underset{CH_3}{CH}-$ | $-COOCH_2CH=CH_2$ | Smp. 40-41°C |
| 224 | H | H | Cl | H | H | H | $-\underset{CH_3}{CH}-$ | $-COOCH_2CH=CH-CH_3$ | Smp. 39-40°C |
| 225 | H | H | Cl | H | H | H | $-\underset{CH_3}{CH}-$ | $-COOCH_2-\overset{CH_3}{C}=CH_2$ | Smp. 62-63°C |
| 226 | H | H | Cl | H | H | H | $-\underset{CH_3}{CH}-$ | $-COO-$ 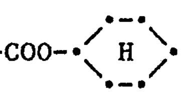 | $n_D^{30}$ = 1,5677 |
| 227 | Br | H | Cl | H | H | H | $-\underset{CH_3}{CH}-$ | $-COO(CH_2)_7CH_3$ | $n_D^{28}$ = 1,5439 |

-39-

0159290

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 228 | Cl | H | Cl | H | H | H | $-CH(CH_3)-$ | $-COOCH(CH_3)(CH_2)_5CH_3$ | $n_D^{25}$ = 1,5408 |
| 229 | Br | H | Cl | H | H | H | $-CH(CH_3)-$ | $-COOCH(CH_3)(CH_2)_5CH_3$ | $n_D^{25}$ = 1,5527 |
| 230 | Br | H | Cl | H | H | H | $-CH(CH_3)-$ | $-COO(CH_2)_{11}CH_3$ | $n_D^{30}$ = 1,5347 |
| 231 | Br | H | Cl | H | H | H | $-CH(CH_3)-$ | $-COOCH_2-$ 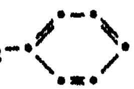 | Smp. 55-56°C |
| 232 | Br | H | Cl | H | H | H | $-CH(CH_3)-$ | $-COOCH_2-$ 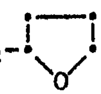 | $n_D^{30}$ = 1,5886 |
| 233 | Br | H | Cl | H | H | H | $-CH(CH_3)-$ | $-COOCH_2CH_2OC_3H_7iso$ | $n_D^{28}$ = 1,5642 |
| 234 | Br | H | Cl | H | H | H | $-CH(CH_3)-$ | $-COOCH_2CH_2O-$ 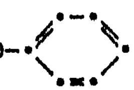 | $n_D^{20}$ = 1,6031 |
| 235 | Br | H | Cl | H | H | H | $-CH(CH_3)-$ | $-COOCH_2CH=CH_2$ | Smp. 55-56°C |
| 236 | Cl | H | Cl | H | H | H | $-CH(CH_3)-$ | $-COOCH_2CH=CH-CH_3$ | Smp. 38-39°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 237 | Br | H | Cl | H | H | H | $-CH(CH_3)-$ | $-COOCH_2CH=CH-CH_3$ | Smp. 38-40°C |
| 238 | Br | H | Cl | H | H | H | $-CH(CH_3)-$ | $-COOCH_2C(CH_3)=CH_2$ | $n_D^{28}$ = 1,5824 |
| 239 | H | H | Cl | H | H | H | $-CH_2-$ | -COO- 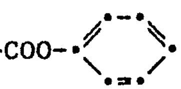 | Smp. 165-170°C |
| 240 | H | H | Cl | H | H | H | $-CH_2-$ | -COO- 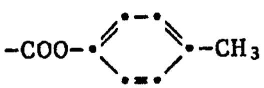 | Smp. 143-145°C |
| 241 | H | H | Cl | H | H | H | $-CH_2-$ | -COO- 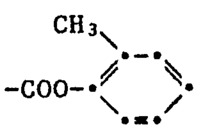 | Smp. 111-116°C |
| 242 | H | H | Cl | H | H | H | $-CH_2-$ | -COO- 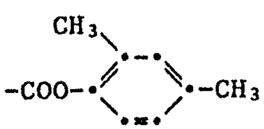 | Smp. 108-119°C |
| 243 | H | H | Cl | H | H | H | $-CH(CH_3)-$ | -COO- 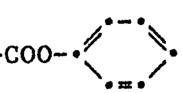 | Smp. 102-105°C |

-41-

0159290

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 244 | H | H | Cl | H | H | H | $-CH_2-$ | $-COO\overset{CH_3}{C}HCH_2CH_2CH_3$ | Smp. 65-70°C |
| 245 | H | H | H | H | H | H | $-CH_2-$ | $-COOCH_2-\overset{CH_3}{C}H(CH_2)_2CH_3$ | Oel; $n_D^{22}$ = 1.5525 |
| 246 | H | H | Cl | H | H | H | $-CH_2-$ | $-COO-$ 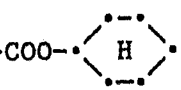 | Smp. 112-113°C |
| 247 | H | H | Cl | H | H | H | $-CH_2-$ | $-COOCH_2\overset{CH_3}{C}H-CH_3$ | Smp. 113-114°C |
| 248 | H | H | H | H | H | H | $-CH_2-$ | $-COO(CH_2)_2\overset{OCH_3}{C}HCH_3$ | Oel; $n_D^{23}$ = 1.5732 |
| 249 | H | H | H | H | H | H | $-CH_2-$ | $-COOCH_2CH_2OCH_2CH_2O(CH_2)_3CH_3$ | Oel; $n_D^{22}$ = 1.5389 |
| 250 | H | H | H | H | H | H | $-CH_2-$ | $-COS(CH_2)_3CH_3$ | Oel; $n_D^{23}$ = 1.6096 |
| 251 | H | H | H | H | H | H | $-CH_2-$ | $-COO-$ 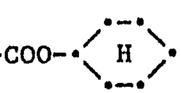 | Oel; $n_D^{23}$ = 1.5755 |
| 252 | H | H | H | H | H | H | $-CH_2-$ | $-COO(CH_2)_4CH_3$ | Oel; $n_D^{23}$ = 1.5591 |
| 253 | H | H | H | H | H | H | $-CH_2-$ | $-COS(CH_2)_7CH_3$ | Oel; $n_D^{22}$ = 1.5697 |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 254 | H | H | Cl | H | H | H | $-CH_2-$ | $-COOCH_2-\overset{CH_3}{C}H(CH_2)_2CH_3$ | Smp. 74-75°C |
| 255 | H | H | Cl | H | H | H | $-CH_2-$ | $-COS(CH_2)_3CH_3$ | Oel; $n_D^{22}$ = 1.6076 |
| 256 | H | H | H | H | H | H | $-CH_2-$ | $-COOCH_2CH=CH-CH_3$ | Oel; $n_D^{22}$ = 1.5833 |
| 257 | H | H | H | H | H | H | $-CH_2-$ | $-COOCH_2-\overset{C_2H_5}{C}H-C_2H_5$ | Oel; $n_D^{23}$ = 1.5530 |
| 258 | H | H | Cl | H | H | H | $-CH_2-$ | $-COOCH_2CH_2OCH_2CH_2O(CH_2)_3CH_3$ | Smp. 39-41°C |
| 259 | H | H | Cl | H | H | H | $-CH_2-$ | $-COO(CH_2)_2\overset{OCH_3}{C}HCH_3$ | Smp. 72-73°C |
| 260 | H | H | Cl | H | H | H | $-CH_2-$ | $-COO(CH_2)_4CH_3$ | Smp. 78-79°C |
| 261 | H | H | Cl | H | H | H | $-CH_2-$ | $-COO\overset{C_2H_5}{C}H-(CH_2)_2CH_3$ | Smp. 37-46°C |
| 262 | H | H | H | H | H | H | $-CH_2-$ | $-COOCH_2CH_2OC_3H_7$-iso | Oel; $n_D^{22}$ = 1.5546 |
| 263 | H | H | Cl | H | H | H | $-CH_2-$ | $-COO(CH_2)_{13}CH_3$ | Smp. 75-76°C |
| 264 | H | H | H | H | H | H | $-CH_2-$ | $-COO\overset{C_2H_5}{C}H-C_2H_5$ | Smp. 47-50°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 265 | H | H | H | H | H | H | $-CH_2-$ | $-COO-$ 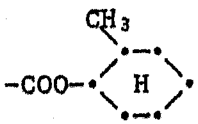 | Smp. 29-31°C |
| 266 | H | H | Cl | H | H | H | $-CH_2-$ | $-COOCH_2-CH(C_2H_5)-C_2H_5$ | Smp. 58-63°C |
| 267 | H | H | H | H | H | H | $-CH_2-$ | $-COOCH_2CH_2OCH_2CH_2OC_2H_5$ | Oel; $n_D^{22}$ = 1.5489 |
| 268 | H | H | H | H | H | H | $-CH_2-$ | $-COOCH_2CH_2O-$ 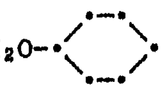 | Smp. 80-81°C |
| 269 | H | H | Cl | H | H | H | $-CH_2-$ | $-COOCH(C_2H_5)-C_2H_5$ | Smp. 55-80°C |
| 270 | H | H | H | H | H | H | $-CH_2-$ | $-COOCH(CH_3)CH_2-CH(CH_3)-CH_3$ | Oel; $n_D^{22}$ = 1.5463 |
| 271 | H | H | H | H | H | H | $-CH_2-$ | $-COO(CH_2)_{13}CH_3$ | Smp. 35-36°C |
| 272 | H | H | H | H | H | H | $-CH_2-$ | $-COOCH_2CH_2O(CH_2)_3CH_3$ | Oel; $n_D^{22}$ = 1.5495 |
| 273 | H | H | Cl | H | H | H | $-CH_2-$ | $-COOCH_2CH_2OCH_2CH_2OC_2H_5$ | Smp. 42-43°C |
| 274 | H | H | H | H | H | H | $-CH_2-$ | $-COOCH_2-CH(CH_3)-C_2H_5$ | Oel; $n_D^{22}$ = 1.5566 |
| 275 | H | H | Cl | H | H | H | $-CH_2-$ | $-COOCH(CH_3)CH_2-CH(CH_3)-CH_3$ | Smp. 63-64°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 276 | H | H | H | H | H | H | $-CH_2-$ | $-COS\overset{CH_3}{C}H-C_2H_5$ | Oel; $n_D^{22}$ = 1.5973 |
| 277 | H | H | Cl | H | H | H | $-CH_2-$ | $-COO-$ 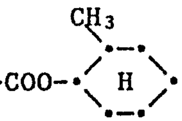 | Smp. 98-101°C |
| 278 | H | H | H | H | H | H | $-CH_2-$ | $-COO\underset{C_2H_5}{\overset{CH_3}{C}}-C_2H_5$ | Oel; $n_D^{22}$ = 1.5551 |
| 279 | H | H | H | H | H | H | $-CH_2-$ | $-COOCH_2-\overset{CH_3}{C}=CH_2$ | Oel; $n_D^{22}$ = 1.5805 |
| 280 | H | H | H | H | H | H | $-CH_2-$ | $-COO\underset{CH_3}{\overset{CH_3}{C}}-CH=CH_2$ | Oel; $n_D^{22}$ = 1.5793 |
| 281 | H | H | H | H | H | H | $-CH_2-$ | $-COOCH_2CH_2OCH_2CH_2OCH_3$ | Oel; $n_D^{23}$ = 1.5560 |
| 282 | H | H | Cl | H | H | H | $-CH_2-$ | $-COO\underset{C_2H_5}{\overset{CH_3}{C}}-C_2H_5$ | Oel; $n_D^{22}$ = 1.5632 |
| 283 | H | H | Cl | H | H | H | $-CH_2-$ | $-COO(CH_2)_{10}CH_3$ | Smp. 70-71°C |
| 284 | H | H | Cl | H | H | H | $-CH_2-$ | $-COOCH_2-\overset{CH_3}{C}H-C_2H_5$ | Smp. 78-79°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 285 | H | H | H | H | H | H | $-CH_2-$ | -COO- 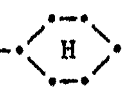 $-CH_3$ | Smp. 40-42°C |
| 286 | H | H | H | H | H | H | $-CH_2-$ | $-COO(CH_2)_6CH_3$ | Oel; $n_D^{23}$ = 1.5469 |
| 287 | H | H | H | H | H | H | $-CH_2-$ | $CH_3$<br>$-COOC-C_2H_5$<br>$CH_3$ | Oel; $n_D^{22}$ = 1.5581 |
| 288 | H | H | Cl | H | H | H | $-CH_2-$ | $-COOCH_2CH_2O(CH_2)_3CH_3$ | Smp. 69-70°C |
| 289 | H | H | Cl | H | H | H | $-CH_2-$ | $CH_3$<br>$-COSCH-C_2H_5$ | Smp. 55-56°C |
| 290 | H | H | Cl | H | H | H | $-CH_2-$ | $CH_3$<br>$-COOC-CH=CH_2$<br>$CH_3$ | Smp. 83-87°C |
| 291 | H | H | H | H | H | H | $-CH_2-$ | $-COSCH_3$ | Smp. 41-44°C |
| 292 | H | H | Cl | H | H | H | $-CH_2-$ | $-COOCH_2CH_2OCH_2CH_2OCH_3$ | Oel; $n_D^{23}$ = 1.5633 |
| 293 | H | H | Cl | H | H | H | $-CH_2-$ | $-COSCH_3$ | Smp. 89-91°C |
| 294 | H | H | Cl | H | H | H | $-CH_2-$ | $CH_3$<br>$-COOC-C_2H_5$<br>$CH_3$ | Smp. 53-54°C |
| 295 | H | H | H | H | H | H | $-CH_2-$ | $-COO(CH_2)_{10}CH_3$ | Oel; $n_D^{23}$ = 1.5310 |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 296 | H | H | Cl | H | H | H | $-CH_2-$ | $-COO(CH_2)_6CH_3$ | Smp. 74-76°C |
| 297 | H | H | H | H | H | H | $-CH_2-$ | $CH_3$ $CH_3$<br>$-COOCH—CH-CH_3$ | Oel; $n_D^{23}$ =1.5554 |
| 298 | H | H | Cl | H | H | H | $-CH_2-$ | -COO- 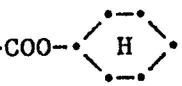 $-CH_3$ | Smp. 103-105°C |
| 299 | H | H | H | H | H | H | $-CH_2-$ | $CH_3$<br>$-COSC-CH_3$<br>$CH_3$ | Oel; $n_D^{23}$ = 1.5987 |
| 300 | H | H | Cl | H | H | H | $-CH_2-$ | $-COS(CH_2)_{11}CH_3$ | Smp. 26-28°C |
| 301 | H | H | Cl | H | H | H | $-CH_2-$ | $-COS(CH_2)_9CH_3$ | Smp. 29-31°C |
| 302 | H | H | Cl | H | H | H | $-CH_2-$ | $-COO(CH_2)_9CH_3$ | Smp. 73-74°C |
| 303 | H | H | H | H | H | H | $-CH_2-$ | $CH_3$<br>$-COOCH(CH_2)_4CH_3$ | Oel; $n_D^{23}$ = 1.5433 |
| 304 | H | H | Cl | H | H | H | $-CH_2-$ | $C_3H_7-n$<br>$-COOCH-C\equiv CH$ | Smp. 81-82°C |
| 305 | H | H | H | H | H | H | $-CH_2-$ | $C_5H_{11}-n$<br>$-COOCH-CH=CH_2$ | Oel; $n_D^{23}$ = 1.5472 |
| 306 | H | H | Cl | H | H | H | $-CH_2-$ | $CH_3$ $CH_3$<br>$-COOCH—CH-CH_3$ | Smp. 70-74°C |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 307 | H | H | Cl | H | H | H | $-CH_2-$ | $-COSC(CH_3)_2-CH_3$ | Oel; $n_D^{22}$ = 1.5996 |
| 308 | H | H | H | H | H | H | $-CH_2-$ | $-COOCH(CH_3)-C\equiv CH$ | Oel; $n_D^{23}$ = 1.5837 |
| 309 | H | H | H | H | H | H | $-CH_2-$ | $-COS(CH_2)_{11}CH_3$ | Oel; $n_D^{23}$ = 1.5523 |
| 310 | H | H | H | H | H | H | $-CH_2-$ | $-COOCH_2-C(CH_3)_2-CH_3$ | Oel; $n_D^{22}$ = 1.5524 |
| 311 | H | H | H | H | H | H | $-CH_2-$ | $-COSC_2H_5$ | Oel; $n_D^{23}$ = 1.6310 |
| 312 | H | H | Cl | H | H | H | $-CH_2-$ | $-COOCH_2-C(CH_3)_2-CH_3$ | Smp. 76-81°C |
| 313 | H | H | Cl | H | H | H | $-CH_2-$ | $-COSC_3H_7-n$ | Oel; $n_D^{22}$ = 1.6136 |
| 314 | H | H | H | H | H | H | $-CH_2-$ | $-COO(CH_2)_9CH_3$ | Oel; $n_D^{22}$ = 1.5308 |
| 315 | H | H | Cl | H | H | H | $-CH_2-$ | $-COOCH(CH_3)(CH_2)_4CH_3$ | Smp. 65-67°C |
| 316 | H | H | H | H | H | H | $-CH_2-$ | $-COO(CH_2)_2CH(CH_3)-CH_3$ | Oel; $n_D^{23}$ = 1.5568 |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 317 | H | H | H | H | H | H | $-CH_2-$ | $-COO\overset{C_2H_5}{\overset{\vert}{C}}H(CH_2)_3CH_3$ | Oel; $n_D^{22}$ = 1.5454 |
| 318 | H | H | Cl | H | H | H | $-CH_2-$ | $-COO(CH_2)_8CH_3$ | Smp. 78-79°C |
| 319 | H | H | H | H | H | H | $-CH_2-$ | $-COSCH_2\overset{CH_3}{\overset{\vert}{C}}HCH_3$ | Oel; $n_D^{23}$ = 1.6049 |
| 320 | H | H | Cl | H | H | H | $-CH_2-$ | $-COSC_2H_5$ | Smp. 55-57°C |
| 321 | H | H | H | H | H | H | $-CH_2-$ | $-COO(CH_2)_8CH_3$ | Oel; $n_D^{24}$ = 1-5436 |
| 322 | H | H | Cl | H | H | H | $-CH_2-$ | $-COOCH_2-\overset{C_2H_5}{\overset{\vert}{C}}H(CH_2)_3CH_3$ | Smp. 45-47°C |
| 323 | H | H | Cl | H | H | H | $-CH_2-$ | $-COSCH_2-\overset{CH_3}{\overset{\vert}{C}}H-CH_3$ | Oel; $n_D^{23}$ = 1.6045 |
| 324 | H | H | H | H | H | H | $-CH_2-$ | $-COS(CH_2)_9CH_3$ | Oel; $n_D^{23}$ = 1.5630 |
| 325 | H | H | Cl | H | H | H | $-CH_2-$ | $-COO(CH_2)_2\overset{CH_3}{\overset{\vert}{C}}H-CH_3$ | Smp. 72-74°C |
| 326 | H | H | Cl | H | H | H | $-CH_2-$ | $-COO\overset{C_2H_5}{\overset{\vert}{C}}H(CH_2)_3CH_3$ | Oel; $n_D^{22}$ = 1.5542 |
| 327 | H | H | H | H | H | H | $-CH_2-$ | $-COO(CH_2)_5CH_3$ | Oel; $n_D^{22}$ = 1.5512 |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 328 | H | H | H | H | H | H | $-CH_2-$ | $-COO\overset{C_3H_7-n}{C}H(CH_2)_2CH_3$ | Smp. 48-50°C |
| 329 | H | H | H | H | H | H | $-CH_2-$ | $-COS(CH_2)_4CH_3$ | Oel; $n_D^{22}$ = 1.5937 |
| 330 | H | H | H | H | H | H | $-CH_2-$ | $-COSC_3H_7$-iso | Oel; $n_D^{23}$ = 1.5821 |
| 331 | H | H | H | H | H | H | $-CH_2-$ | $-COOCH_2\overset{C_2H_5}{C}H-(CH_2)_3CH_3$ | Oel; $n_D^{22}$ = 1.5395 |
| 332 | H | H | Cl | H | H | H | $-CH_2-$ | $-COO\overset{C_3H_7-n}{C}H(CH_2)_2CH_3$ | Smp. 55-57°C |
| 333 | H | H | Cl | H | H | H | $-CH_2-$ | $-COS(CH_2)_5CH_3$ | Oel; $n_D^{22}$ = 1.5882 |
| 334 | H | H | Cl | H | H | H | $-CH_2-$ | $-COS(CH_2)_4CH_3$ | Oel; $n_D^{23}$ = 1.5990 |
| 335 | H | H | Cl | H | H | H | $-CH_2-$ | $-COO(CH_2)_5CH_3$ | Smp. 71-72°C |
| 336 | H | H | Cl | H | H | H | $-CH_2-$ | $-COSC_3H_7$-iso | Smp. 62-64°C |
| 337 | H | H | Cl | H | H | H | $-CH_2-$ | $-COO\overset{C_2H_5}{C}H-CH_2\overset{CH_3}{C}HC_2H_5$ | Smp. 25-29°C |
| 338 | H | H | H | H | H | H | $-CH_2-$ | $-COO\overset{C_3H_7-iso}{C}H-C_3H_7iso$ | Oel; $n_D^{22}$ = 1.5468 |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 339 | H | H | H | H | H | H | $-CH_2-$ | $CH_3$<br>$-COOCH-(CH_2)_3CH_3$ | Oel; $n_D^{23} = 1.5531$ |
| 340 | H | H | Cl | H | H | H | $-CH_2-$ | $C_5H_{11}-n$<br>$-COOCH-CH=CH_2$ | Oel; $n_D^{23} = 1.5579$ |
| 341 | H | H | H | H | H | H | $-CH_2-$ | $C_2H_5$<br>$-COOCH-(CH_2)_2CH_3$ | Smp. 42-44°C |
| 342 | H | H | H | H | H | H | $-CH_2-$ | $-COSC_3H_7-n$ | Oel; $n_D^{22} = 1.6108$ |
| 343 | H | H | Cl | H | H | H | $-CH_2-$ | $CH_3$<br>$-COOCH-(CH_2)_3CH_3$ | Smp. 68-71°C |
| 343 | H | H | Cl | H | H | H | $-CH_2-$ | $CH_3$<br>$-COOCH-(CH_2)_3CH_3$ | Smp. 68-71°C |
| 344 | H | H | H | H | H | H | $-CH_2-$ | $C_2H_5$ $CH_3$<br>$-COOCHCH_2CHC_2H_5$ | Oel; $n_D^{23} = 1.5472$ |
| 345 | H | H | Cl | H | H | H | $-CH_2-$ | $C_3H_7-iso$<br>$-COOCH-C_3H_7-iso$ | Smp. 88-89°C |
| 346 | H | H | H | H | H | H | $-CH_2-$ | $-COS(CH_2)_5CH_3$ | Oel; $n_D^{22} = 1.5804$ |
| 347 | H | H | H | H | H | H | $-CH_2-$ | $-COO(CH_2)_9-CH=CH_2$ | Oel; $n_D^{22} = 1.5386$ |

Tabelle 1 (Fortsetzung)

| Nr. | $R_1$ | $R_2$ | $R_3$ | $R_4$ | $R_5$ | $R_6$ | A | Z | physikal. Konstante |
|---|---|---|---|---|---|---|---|---|---|
| 348 | H | H | H | H | H | H | $-CH_2-$ | $C_3H_7$-n<br>$-COOCH-C{\equiv}CH$ | Oel; $n_D^{22}$ = 1.5659 |
| 349 | H | H | Cl | H | H | H | $-CH_2-$ | $CH_3$<br>$-COOCH-C{\equiv}CH$ | Smp. 97-100°C |
| 350 | H | H | H | H | H | H | $-CH_2-$ | $CH_3$<br>$-COOC-C{\equiv}CH$<br>$C_2H_5$ | Oel; $n_D^{22}$ = 1.5688 |
| 351 | H | H | Cl | H | H | H | $-CH_2-$ | $-COO(CH_2)_9-CH{=}CH_2$ | Smp. 66-67°C |
| 352 | H | H | Cl | H | H | H | $-CH_2-$ | $CH_3$<br>$-COO-C-C{\equiv}CH$<br>$CH_3$ | Smp. 76-81°C |
| 353 | H | H | H | H | H | H | $-CH_2-$ | $CH_3$<br>$-COO-C-C{\equiv}CH$<br>$CH_3$ | Oel; $n_D^{23}$ = 1.5740 |
| 354 | H | H | Cl | H | H | H | $-CH_2-$ | $CH_3$<br>$-COO-C-C{\equiv}CH$<br>$C_2H_5$ | Smp. 78-79°C |
| 355 | H | H | Cl | H | H | H | $-CH_2-$ | $CH_3$ $CH_3$<br>$-COO-CH-CH-C_2H_5$ | Smp. 71-73°C |

Tabelle 1 (Fortsetzung)

| Nr. | $R_1$ | $R_2$ | $R_3$ | $R_4$ | $R_5$ | $R_6$ | A + Z | physikal. Konstante |
|---|---|---|---|---|---|---|---|---|
| 356 | H | H | Cl | H | H | H | 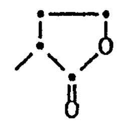 | Smp. 140-141,5°C |

Die Verbindungen der Formel I lassen sich nach bekannten Methoden herstellen, wie sie beispielsweise in den europäischen Patentpublikationen 86 750 und 94 349 beschrieben sind, oder sind analog bekannten Methoden herstellbar.

Die Chinolinderivate der Formel I besitzen in hervorragendem Masse die Eigenschaften, Kulturpflanzen gegen schädigende Wirkungen von herbizid wirksamen Derivaten von 2-[4-(Benzoxazol-, Benzthiazol- und Chinoxalin-2-yl-oxy)-phenoxy]-propionsäure-estern zu schützen. Die vorgenannten herbiziden Derivate entsprechen der Formel II

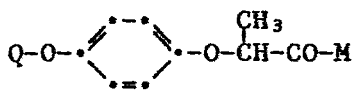 (II),

worin M für -OR' oder $-N(CN)-R'_2$ , R' für $C_1$-$C_{12}$-Alkyl, welches durch 1 bis 6 Halogenatome, durch $C_1$-$C_6$-Alkoxy oder $C_1$-$C_4$-Alkylthio substituiert ist, $R'_2$ für $C_1$-$C_4$-Alkyl, $C_3$-$C_4$-Alkenyl, $C_3$-$C_4$-Alkinyl oder Alkoxyalkyl mit insgesamt 2 bis 4 Kohlenstoffatomen, und Q für

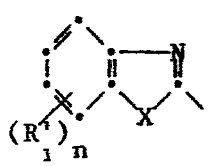 oder 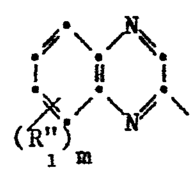 ,

worin X ein Sauerstoff- oder Schwefelatom, $R'_1$ und $R''_2$ Halogen, Trifluormethyl, Nitro, Cyano, $C_1$-$C_4$-Alkyl oder $C_1$-$C_4$-Alkoxy und n und m 0, 1 oder 2 bedeuten, oder, wenn M $-N(CN)-R'_2$ bedeutet, zusätzlich für

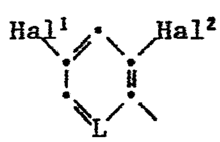 ,

worin $Hal^1$ Fluor, Chlor, Brom, Jod oder Trifluormethyl, $Hal^2$ Fluor, Chlor, Brom, Trifluormethyl oder Wasserstoff und L das Strukturelement =N- oder =CH- bedeuten, steht.

In den Verbindungen der Formel II bedeutet Halogen Fluor, Chlor, Brom und Jod, vorzugsweise Fluor, Chlor und Brom.

Unter Alkyl als Substituent oder Teil eines Substituenten sind alle geradkettigen und alle verzweigten Alkylgruppen mit der entsprechenden Anzahl an Kohlenstoffatomen zu verstehen. So steht beispielsweise $C_1$-$C_4$-Alkyl für Methyl, Ethyl, n-Propyl, Isopropyl, n-Butyl, sek.-Butyl, tert.-Butyl und Isobutyl. Diese Alkylgruppen liegen auch in den $C_1$-$C_4$-Alkoxygruppen vor.

Herbizid wirksame Verbindungen der Formel II sind beispielsweise in den deutschen Offenlegungsschriften 26 40 730 und 30 04 770 beschrieben.

Besonders bemerkenwert ist die Schutzwirkung von Chinolinderivaten der Formel I gegenüber solchen Herbiziden der Formel II, in denen R' die für Formel II angegebene Bedeutung hat und Q für 6-Halogenbenzoxazol-2-yl, 6-Halogenbenzothiazol-2-yl oder 6-Halogenchinoxalin-2-yl steht, und vor allem gegenüber 2-[4-(6-Chlorbenzoxazol-2-yloxy)-phenoxy]-propionsäure-ethylester, 2-[4-(6-Chlorbenzothiazol-2-yl-oxy)-phenoxy]-propionsäure-ethylester und 2-[4-(6-Chlorchinoxalin-2-yl-oxy)-phenoxy]-propionsäure-ethylester.

Als Kulturpflanzen, welche durch Chinolinderivate der Formel I gegen schädigende Wirkungen von Herbiziden der Formel II geschützt werden können, kommen insbesondere diejenigen in Betracht, die auf dem Nahrungs- oder Textilsektor von Bedeutung sind, beispielsweise Baumwolle, Zuckerrüben, Zuckerrohr und insbesondere Kulturhirse, Mais, Reis und andere Getreidearten (Weizen, Roggen, Gerste, Hafer).

Ein geeignetes Verfahren zum Schützen von Kulturpflanzen unter Verwendung von Verbindungen der Formel I besteht darin, dass man Kulturpflanzen, Teile dieser Pflanzen oder für den Anbau der Kulturpflanzen bestimmte Böden vor oder nach dem Einbringen des pflanzlichen Materials in den Boden mit einer Verbindung der Formel I oder einem Mittel, welches eine solche Verbindung enthält, behandelt. Die Behandlung kann vor, gleichzeitig mit oder nach dem Einsatz des

Herbizids der Formel II erfolgen. Als Pflanzenteile kommen insbesondere diejenigen in Betracht, die zur Neubildung einer Pflanze befähigt sind, wie beispielsweise Samen, Früchte, Stengelteile und Zweige (Stecklinge) sowie auch Wurzeln, Knollen und Rhizome.

Die Erfindung betrifft auch ein Verfahren zur selektiven Bekämpfung von Unkräutern in Kulturpflanzenbeständen, wobei die Kulturpflanzenbestände, Teile der Kulturpflanzen oder Anbauflächen für Kulturpflanzen mit einem Herbizid der Formel II und einer Verbindung der Formel I oder einem Mittel, welches eine Kombination aus einem solchen Herbizid und einer Verbindung der Formel I enthält, behandelt werden.

Bei den zu bekämpfenden Unkräutern kann es sich sowohl um monokotyle wie um dikotyle Unkräuter handeln.

Als Kulturpflanzen oder Teile dieser Pflanzen kommen beispielsweise die vorstehend genannten in Betracht. Als Anbauflächen gelten die bereits mit den Kulturpflanzen bewachsenen oder mit dem Saatgut dieser Kulturpflanzen beschickten Bodenareale, wie auch die zur Bebauung mit diesen Kulturpflanzen bestimmten Böden.

Die zu applizierende Aufwandmenge Antidot im Verhältnis zum Herbizid richtet sich weitgehend nach der Anwendungsart. Bei einer Feldbehandlung, welche entweder unter Verwendung einer Tankmischung mit einer Kombination von Antidot und Herbizid oder durch getrennte Applikation von Antidot und Herbizid erfolgt, liegt in der Regel ein Verhältnis von Antidot zu Herbizid von 1:100 bis 10:1, bevorzugt 1:5 bis 8:1, und insbesondere 1:1, vor. Dagegen werden bei der Samenbeizung weit geringere Mengen Antidot im Verhältnis zur Aufwandmenge an Herbizid pro Hektar Anbaufläche benötigt.

In der Regel werden bei der Feldbehandlung 0,1 bis 10 kg Antidot/ha, vorzugsweise 0,5 bis 2 kg Antidot/ha, appliziert,

Bei der Samenbeizung werden im allgemeinen 0,01 bis 10 g Antidot/kg Samen, vorzugsweise 0,05 bis 1 g Antidot/kg Samen, appliziert. Wird das Antidot in flüssiger Form kurz vor der Aussaat unter Samenquellung appliziert, so werden zweckmässigerweise Antidot-Lösungen verwendet, welche den Wirkstoff in einer Konzentration von 1 bis 10 000, vorzugsweise von 100 bis 1 000 ppm, enthalten.

Zur Applikation werden die Verbindungen der Formel I oder Kombinationen von Verbindungen der Formel I mit den zu antagonisierenden Herbiziden zweckmässigerweise zusammen mit den in der Formulierungstechnik üblichen Hilfsmitteln eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen, Bestreichen oder Giessen werden gleich wie die Art der zu verwendenden Mittel den angestrebten Zielen und den gegebenen Verhältnissen entsprechend gewählt.

Die Formulierungen, d.h. die den Wirkstoff der Formel I oder eine Kombination von Wirkstoff der Formel I mit zu antagonisierendem Herbizid und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmiteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether,

Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffs der Formel I und gegebenenfalls auch dem zu antagonisierenden Herbizid nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood New Jersey, 1981
Stache, H., "Tensid-Taschenbuch",
Carl Hanser Verlag, München/Wien, 1981

Die agrochemischen Zubereitungen enthalten in der Regel 0,1 bis 99 Gewichtsprozent, insbesondere 0,1 bis 95 Gew.-%, Wirkstoff der Formel I oder Wirkstoffgemisch Antidot/Herbizid, 1 bis 99,9 Gew.-%, insbesondere 5 bis 99,8 Gew.-%, eines festen oder flüssigen Zusatzstoffes und 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Für die Verwendung von Verbindungen der Formel I oder sie enthaltender Mittel zum Schützen von Kulturpflanzen gegen schädigende Wirkungen von Herbiziden der Formel II kommen verschiedene Methoden und Techniken in Betracht, wie beispielsweise die folgenden:

i) Samenbeizung

a) Beizung der Samen mit einem als Spritzpulver formulierten Wirkstoff der Formel I durch Schütteln in einem Gefäss bis zur gleichmässigen Verteilung auf der Samenoberfläche (Trockenbeizung). Man verwendet dabei etwa 1 bis 500 g Wirkstoff der Formel I (4 g bis 2 kg Spritzpulver) pro 100 kg Saatgut.

b) Beizung der Samen mit einem Emulsionskonzentrat des Wirkstoffs der Formel I nach der Methode a) (Nassbeizung).

c) Beizung durch Tauchen des Saatguts in eine Brühe mit 50-3200 ppm Wirkstoff der Formel I während 1 bis 72 Stunden und gegebenenfalls nachfolgendes Trocknen der Samen (Tauchbeizung).

Die Beizung des Saatguts oder die Behandlung des angekeimten Sämlings sind naturgemäss die bevorzugten Methoden der Applikation, weil die Wirkstoffbehandlung vollständig auf die Zielkultur gerichtet ist. Man verwendet in der Regel 1 bis 500 g Antidot, vorzugsweise 5 bis 250 g Antidot, pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) Applikation aus Tankmischung

Eine flüssige Aufarbeitung eines Gemisches von Antidot und Herbizid (gegenseitiges Mengenverhältnis zwischen 10:1 und 1:10) wird verwendet, wobei die Aufwandmenge an Herbizid 0,1 bis 10 kg pro Hektar

beträgt. Solche Tankmischung wird vorzugsweise vor oder unmittelbar nach der Aussaat appliziert oder 5 bis 10 cm tief in den noch nicht gesäten Boden eingearbeitet.

iii) Applikation in der Saatfurche

Das Antidot wird als Emulsionskonzentrat, Spritzpulver oder als Granulat in die offene besäte Saatfurche eingebracht und hierauf wird nach dem Decken der Saatfurche in normaler Weise das Herbizid im Vorauflaufverfahren appliziert.

iv) Kontrollierte Wirkstoffabgabe

Der Wirkstoff der Formel I wird in Lösung auf mineralische Granulatträger oder polymerisierte Granulate (Harnstoff/Formaldehyd) aufgezogen und trocknen gelassen. Gegebenenfalls kann ein Ueberzug aufgebracht werden (Umhüllungsgranulate), der es erlaubt, den Wirkstoff über einen bestimmten Zeitraum dosiert abzugeben.

Formulierungsbeispiel für flüssige Wirkstoffe der Formel I
(% = Gewichtsprozent)

| 1. Emulsions-Konzentrate | a) | b) | c) |
|---|---|---|---|
| Wirkstoff aus Tabelle 1 | 25 % | 40 % | 50 % |
| Ca-Dodecylbenzolsulfonat | 5 % | 8 % | 6 % |
| Ricinusöl-polyethylenglykolether (36 Mol AeO) | 5 % | - | - |
| Tributylphenol-polyethylenglykolether (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff aus Tabelle 1 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°) | - | - | 94 % | - |

Die Lösungen sind zur Anwendung in Form kleinster Tropfen geeignet.

| 3. Granulate | a) | b) |
|---|---|---|
| Wirkstoff aus Tabelle 1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmitel anschliessend im Vakuum abgedampft.

| 4. Stäubemittel | a) | b) |
|---|---|---|
| Wirkstoff aus Tabelle 1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemitel.

Formulierungsbeispiele für feste Wirkstoffe der Formel I

(% = Gewichtsprozent)

| 5. Spritzpulver | a) | b) | c) |
|---|---|---|---|
| Wirkstoff aus Tabelle 1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol AeO) | - | 2 % | - |
| Hochdisperse Kieselsäure | 5 % | 10 % | 10 % |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 6. Emulsions-Konzentrate | |
|---|---|
| Wirkstoff aus Tabelle 1 | 10 % |
| Octylphenolpolyethylenglykolether (4-5 Mol AeO) | 3 % |
| Ca-Dodecylbenzolsulfonat | 3 % |
| Ricinusölpolyglykolether (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff aus Tabelle 1 | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

8. Extruder-Granulate

| | |
|---|---|
| Wirkstoff aus Tabelle 1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

9. Umhüllungs-Granulate

| | |
|---|---|
| Wirkstoff aus Tabelle 1 | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

10. Suspensions-Konzentrate

| | |
|---|---|
| Wirkstoff aus Tabelle 1 | 40 % |
| Ethylenglykol | 10 % |
| Nonylphenolpolyethylenglykolether (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der feingemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Formulierungsbeispiele für Wirkstoffgemische (flüssig

(% = Gewichtsprozent)

| 11. Emulsions-Konzentrate | a) | b) | c) |
|---|---|---|---|
| Wirkstoffgemisch:Antidot aus Tabelle 1 und ein Herbizid der Formel II im Verhältnis 1:1 | 25 % | 40 % | 50 % |
| Ca-Dodecylbenzolsulfonat | 5 % | 8 % | 6 % |
| Ricinusöl-polyethylenglykolether (36 Mol AeO) | 5 % | - | - |
| Tributylphenol-polyethylenglykolether (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 12. Emulsions-Konzentrate | a) | b) | c) |
|---|---|---|---|
| Wirkstoffgemisch: Antidot aus Tabelle 1 und ein Herbizid der Formel II im Verhältnis 1:3 | 25 % | 40 % | 50 % |
| Ca-Dodecylbenzolsulfonat | 5 % | 8 % | 6 % |
| Ricinusöl-polyethylenglykolether (36 Mol AeO) | 5 % | - | - |
| Tributylphenol-polyethylenglykolether (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 13. Emulsions-Konzentrate | a) | b) | c) |
|---|---|---|---|
| Wirkstoffgemisch: Antidot aus Tabelle 1 und ein Herbizid der Formel II im Verhältnis 2:1 | 25 % | 40 % | 50 % |
| Ca-Dodecylbenzolsulfonat | 5 % | 8 % | 6 % |
| Ricinusöl-polyethylenglykolether (36 Mol AeO) | 5 % | - | - |
| Tributylphenol-polyethylenglykolether (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 14. Emulsions-Konzentrate | a) | b) | c) |
|---|---|---|---|
| Wirkstoffgemisch: Antidot aus Tabelle 1 und 2-[4-(6-Chlorchinoxalin-2-yl-oxy)-phenoxy]-propionsäure-ethylester im Verhältnis 1:1 | 25 % | 40 % | 50 % |
| Ca-Dodecylbenzolsulfonat | 5 % | 8 % | 6 % |
| Ricinusöl-polyethylenglykolether (36 Mol AeO) | 5 % | - | - |
| Tributylphenol-polyethylenglykolether (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 15. Emulsions-Konzentrat | a) | b) | c) |
|---|---|---|---|
| Wirkstoffgemisch: Antidot aus Tabelle 1 und 2-[4-(6-Chlorbenzoxazol-2-yl-oxy)-phenoxy]-propionsäure-ethylester im Verhältnis 1:3 | 25 % | 40 % | 50 % |
| Ca-Dodecylbenzolsulfonat | 5 % | 8 % | 6 % |
| Ricinusöl-polyethylenglykolether (36 Mol AeO) | 5 % | - | - |
| Tributylphenol-polyethylenglykolether (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 16. Lösungen | a) | b) | c) | d) |
|---|---|---|---|---|
| Wirkstoffgemisch: Antidot aus Tabelle 1 und ein Herbizid der Formel II im Verhältnis 1:4 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 17. Lösungen | a) | b) | c) | d) |
|---|---|---|---|---|
| Wirkstoffgemisch: Antidot aus Tabelle 1 und ein Herbizid der Formel II im Verhältnis 5:2 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 18. Lösungen | a) | b) | c) | d) |
| --- | --- | --- | --- | --- |
| Wirkstoffgemisch: Antidot aus Tabelle 1 und ein Herbizid der Formel II im Verhältnis 1:1 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 19. Lösungen | a) | b) | c) | d) |
| --- | --- | --- | --- | --- |
| Wirkstoffgemisch: Antidot aus Tabelle 1 und 2-[4-(6-Chlor-benzothiazol-2-yl-oxy)-phenoxy]-propionsäure-ethylester im Verhältnis 1:1 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 20. Lösungen | a) | b) | c) | d) |
| --- | --- | --- | --- | --- |
| Wirkstoffgemisch: Antidot aus Tabelle 1 und 2-[4-(6-Chlorchinoxalin-2-yl-oxy)-phenoxy]-propionsäure-ethylester im Verhältnis 1:4 | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| 21. Granulate | a) | b) |
|---|---|---|
| Wirkstoffgemisch: Antidot aus Tabelle 1 und ein Herbizid der Formel II im Verhältnis 1:1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 22. Granulate | a) | b) |
|---|---|---|
| Wirkstoffgemisch: Antidot aus Tabelle 1 und 2-[4-(6-Chlorbenzoxazol-2-yl-oxy)-phenoxy]-propionsäure-ethylester im Verhältnis 1:1 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| 23. Stäubemittel | a) | b) |
|---|---|---|
| Wirkstoffgemisch: Antidot aus Tabelle 1 und ein Herbizid der Formel II im Verhältnis 1:1 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemitel.

Formulierungsbeispiele für Wirkstoffgemische (fest)

(% = Gewichtsprozent)

24. Spritzpulver

| | a) | b) | c) |
|---|---|---|---|
| Wirkstoffgemisch: Antidot aus Tabelle 1 und ein Herbizid der Formel II im Verhältnis 1:1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

25. Spritzpulver

| | a) | b) | c) |
|---|---|---|---|
| Wirkstoffgemisch: Antidot aus Tabelle 1 und ein Herbizid der Formel II im Verhältnis 1:4 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

26. Spritzpulver

| | a) | b) | c) |
|---|---|---|---|
| Wirkstoffgemisch: Antidot aus Tabelle 1 und ein Herbizid der Formel II im Verhältnis 3:1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykolether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

27. Emulsions-Konzentrate

| | |
|---|---|
| Wirkstoffgemisch: Antidot aus Tabelle 1 und ein Herbizid der Formel II im Verhältnis 1:1 | 10 % |
| Octylphenolpolyethylenglykolether (4-5 Mol AeO) | 3 % |
| Ca-Dodecylbenzolsulfonat | 3 % |
| Ricinusölpolyglykolether (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

28. Emulsions-Konzentrate

| | |
|---|---|
| Wirkstoffgemisch: Antidot aus Tabelle 1 und ein Herbizid der Formel II im Verhältnis 5:2 | 10 % |
| Octylphenolpolyethylenglykolether (4-5 Mol AeO) | 3 % |
| Ca-Dodecylbenzolsulfonat | 3 % |
| Ricinusölpolyglykolether (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

29. Emulsions-Konzentrate

| | |
|---|---|
| Wirkstoffgemisch: Antidot aus Tabelle 1 und ein Herbizid der Formel II im Verhältnis 1:4 | 10 % |
| Octylphenolpolyethylenglykolether (4-5 Mol AeO) | 3 % |
| Ca-Dodecylbenzolsulfonat | 3 % |
| Ricinusölpolyglykolether (35 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| 30. Stäubemittel | a) | b) |
|---|---|---|
| Wirkstoffgemisch: Antidot aus Tabelle 1 und ein Herbizid der Formel II im Verhältnis 1:1 | 5 % | 8 % |
| Talkum | 95 % | - |
| Kaolin | - | 92 % |

Man erhält anwendungsfertige Stäubemittel, indem der Wirkstoff mit den Trägerstoffen vermischt und auf einer geeigneten Mühle vermahlen wird.

31. Extruder-Granulate

| | |
|---|---|
| Wirkstoffgemisch: Antidot aus Tabelle 1 und ein Herbizid der Formel II im Verhältnis 1:1 | 10 % |
| Na-Liginsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

32. Umhüllungs-Granulate

| | |
|---|---|
| Wirkstoffgemisch: Antidot aus Tabelle 1 und ein Herbizid der Formel II im Verhältnis 1:1 | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

33. Suspensions-Konzentrate

| | |
|---|---|
| Wirkstoffgemisch: Antidot aus Tabelle 1 und ein Herbizid der Formel II im Verhältnis 1:1 | 40 % |
| Ethylenglykol | 10 % |
| Nonylphenolpolyethylenglykolether (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

34. Suspensions-Konzentrate

| | |
|---|---|
| Wirkstoffgemisch: Antidot aus Tabelle 1 und ein Herbizid der Formel II im Verhältnis 1:4 | 40 % |
| Ethylenglykol | 10 % |
| Nonylphenolpolyethylenglykolether (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

35. Suspensions-Konzentrate

| | |
|---|---|
| Wirkstoffgemisch: Antidot aus Tabelle 1 und ein Herbizid der Formel II im Verhältnis 3:1 | 40 % |
| Ethylenglykol | 10 % |
| Nonylphenolpolyethylenglykolether (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Biologische Beispiele

### Testbeschreibung

Im Gewächshaus werden Plastiktöpfe, welche 0,5 l Erde enthalten, mit Samen der zu testenden Pflanzen beschickt. Wenn die Pflanzen das 2- bis 3-Blattstadium erreicht haben, werden ein Safener der Formel I und ein Herbizid der Formel II zusammen als Tankmischung appliziert. 21 Tage nach der Applikation wird die Schutzwirkung des Safeners in Prozent bonitiert. Als Referenz dienen dabei mit dem Herbizid allein behandelte Pflanzen sowie die vollständig unbehandelte Kontrolle. Die Resultate sind in den nachfolgenden Tabellen dargestellt. In diesen Tabellen bedeuten

A = 2-[4-(6-Chlorbenzoxazol-2-yl-oxy)-phenoxy]-propionsäureethylester

B = 2-[4-(6-Chlorchinoxalin-2-yl-oxy)-phenoxy]-propionsäureethylester

C = 2-[4-(6-Chlorbenzothiazol-2-yl-oxy)-phenoxy]-propionsäure-ethylester

Ge = Gerste der Sorte "Cornel"

We = Weizen der Sorte "Besso"

Av = Avena fatua (Unkraut)

Tabelle 2

| Testpflanze | Safener Verbindung Nr. 13, kg AS/ha | Herbizid A kg AS/ha | Relative Schutzwirkung in % |
|---|---|---|---|
| Ge | 1,0 | 1,0 | 37,5 |
| Ge | 0,5 | 1,0 | 37,5 |
| Ge | 0,5 | 0,5 | 12,5 |
| Ge | 0,25 | 0,5 | 12,5 |

Formulierung Safener: 25 %iges Spritzpulver

Formulierung Herbizid: 25 %iges Emulsionskonzentrat

Tabelle 3

| Testpflanze | Safener Verbindung Nr.186, kg AS/ha | Herbizid B kg AS/ha | Relative Schutzwirkung in % |
|---|---|---|---|
| Ge | 0,062 | 0,062 | 12,5 |
| Ge | 0,031 | 0,062 | 37,5 |
| Ge | 0,125 | 0,125 | 12,5 |
| Ge | 0,0625 | 0,125 | 12,5 |

Formulierung Safener: 10 %iges Emulsionskonzentrat

Formulierung Herbizid: 25 %iges Emulsionskonzentrat

Tabelle 4

| Test-pflanze | Safener Verbindung Nr.186, kg AS/ha | Herbizid A kg AS/ha | Relative Schutzwirkung in % |
|---|---|---|---|
| Ge | 0,5 | 0,5 | 25 |
| Ge | 0,25 | 0,5 | 25 |
| Ge | 1,0 | 1,0 | 50 |
| Ge | 0,5 | 1,0 | 62,5 |
| Av | 0,5 | 0,5 | 0 |
| Av | 0,25 | 0,5 | 12,5 |
| Av | 1,0 | 1,0 | 0 |
| Av | 0,5 | 1,0 | 0 |

Formulierung Safener: 10 %iges Emulsionskonzentrat

Formulierung Herbizid: 25 %iges Emulsionskonzentrat

Tabelle 5

| Test-pflanze | Safener Verbindung Nr.186, kg AS/ha | Herbizid C kg AS/ha | Relative Schutzwirkung in % |
|---|---|---|---|
| We | 0,5 | 0,5 | 87,5 |
| We | 0,25 | 0,5 | 87,5 |
| We | 1,0 | 1,0 | 75 |
| We | 0,5 | 1,0 | 75 |
| We | 2,0 | 2,0 | 37,5 |
| We | 1,0 | 2,0 | 12,5 |
| Ge | 0,5 | 0,5 | 75 |
| Ge | 0,25 | 0,5 | 75 |
| Ge | 1,0 | 1,0 | 50 |
| Ge | 0,5 | 1,0 | 62,5 |
| Ge | 2,0 | 2,0 | 25 |
| Ge | 1,0 | 2,0 | 12,5 |

Formulierung Safener: 10 %iges Emulsionskonzentrat

Formulierung Herbizid: 25 %iges Emulsionskonzentrat

Patentansprüche

1. Verfahren zum Schützen von Kulturpflanzen gegen schädigende Wirkungen herbizid wirksamer Derivate von 2-[4-Benzoxazol-, Benzthiazol- und Chinoxalin-2-yl-oxy)-phenoxy]-propionsäure-estern der Formel II

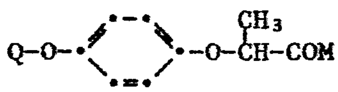

(II),

worin M für -OR' oder $-N(CN)-R'_2$, R' für $C_1-C_{12}$-Alkyl, welches durch 1 bis 6 Halogenatome, durch $C_1-C_6$-Alkoxy oder $C_1-C_4$-Alkylthio substituiert ist, $R'_2$ für $C_1-C_4$-Alkyl, $C_3-C_4$-Alkenyl, $C_3-C_4$-Alkinyl oder Alkoxyalkyl mit insgesamt 2 bis 4 Kohlenstoffatomen, und Q für

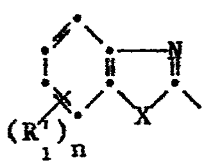

oder

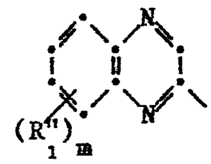

,

worin X ein Sauerstoff- oder Schwefelatom, $R'_1$ und $R''_1$ Halogen, Trifluormethyl, Nitro, Cyano, $C_1-C_4$-Alkyl oder $C_1-C_4$-Alkoxy und n und m 0, 1 oder 2 bedeuten, oder, wenn M $-N(CN)-R'_2$ bedeutet, zusätzlich für

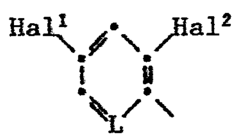

,

worin $Hal^1$ Fluor, Chlor, Brom. Jod oder Trifluormethyl, $Hal^2$ Fluor, Chlor, Brom, Trifluormethyl oder Wasserstoff und L das Strukturelement =N- oder =CH- bedeuten, steht, dadurch gekennzeichnet, dass man die Kulturpflanzen, Teile dieser Pflanzen oder für den Anbau der Kulturpflanzen bestimmte Böden mit einer Verbindung der Formel I

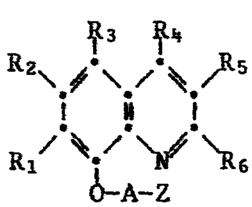

(I),

worin $R_1$, $R_2$ und $R_3$ unabhängig voneinander Wasserstoff, Halogen, Nitro, Cyano, $C_1$-$C_3$-Alkyl oder $C_1$-$C_3$-Alkoxy,
$R_4$, $R_5$ und $R_6$ unabhängig voneinander Wasserstoff, Halogen oder $C_1$-$C_3$-Alkyl,
A eine der Gruppen $-CH_2-$, $-CH_2-CH_2-$ oder $-CH(CH_3)-$ und
Z Cyan oder Amidoxim, welches am Sauerstoffatom acyliert sein kann, eine Carboxylgruppe oder ein Salz davon, eine Mercaptocarbonylgruppe oder ein Salz davon, eine Carbonsäureestergruppe, eine Carbonsäurethiolestergruppe, eine unsubstituierte oder substituierte Carbonsäureamidgruppe, ein cyclisiertes, unsubstituiertes oder substituiertes Derivat einer Carbonsäureamidgruppe oder eine Carbonsäurehydrazidgruppe oder
A und Z zusammen einen unsubstituierten oder substituierten Tetrahydrofuran-2-on-Ring bedeuten, unter Einschluss ihrer Säureadditionssalze und Metallkomplexe, oder einem Mittel, welches eine dieser Verbindungen enthält, behandelt.

2. Verfahren nach Anspruch 1, dadurch gekennzeichnet, dass man eine Verbindung der Formel I, in welcher $R_1$, $R_2$ und $R_3$ unabhängig voneinander Wasserstoff, Halogen, Nitro, Cyan, $C_1$-$C_3$-Alkyl oder $C_1$-$C_3$-Alkoxy,
$R_4$, $R_5$ und $R_6$ unabhängig voneinander Wasserstoff, Halogen oder $C_1$-$C_3$-Alkyl,
A eine der Gruppen $-CH_2-$, $-CH_2-CH_2-$ oder $-CH(CH_3)-$ und
Z Cyan, eine der Gruppen

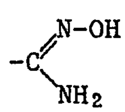

oder

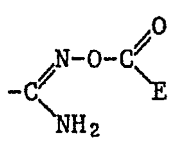

,

einen gegebenenfalls substituierten Oxazolin-2-yl-Rest, $-COOR_{12}$, $-COSR_{13}$ oder $-CONR_{14}R_{15}$ bedeuten, worin
E für $-R_7$, $-OR_8$, $-SR_9$ oder $-NR_{10}R_{11}$ steht, worin
$R_7$ $C_1$-$C_7$-Alkyl, welches unsubstituiert oder durch Halogen oder $C_1$-$C_4$-Alkoxy substituiert ist, $C_3$-$C_6$-Cycloalkyl, $C_2$-$C_4$-Alkenyl, Phenyl, welches unsubstituiert oder durch Halogen, Nitro oder $C_1$-$C_3$-Alkyl

substituiert ist, Benzyl, welches unsubstituiert oder durch Halogen, Nitro oder $C_1$-$C_3$-Alkyl substituiert ist, oder einen 5-bis 6-gliedrigen heterocyclischen Ring, welcher ein oder zwei Heteroatome aus der Gruppe N, O und S enthält und unsubstituiert oder durch Halogen substituiert ist,
$R_8$, $R_9$ und $R_{10}$ unabhängig voneinander $C_1$-$C_8$-Alkyl, welches unsubstituiert oder durch Halogen substituiert ist, $C_2$-$C_4$-Alkenyl, $C_3$-$C_6$-Alkinyl, Phenyl, welches unsubstituiert oder durch Halogen, $C_1$-$C_3$-Alkyl, $C_1$-$C_3$-Alkoxy, Trifluormethyl oder Nitro substituiert ist, oder Benzyl, welches unsubstituiert oder durch Halogen oder Nitro substituiert ist,
$R_{11}$ Wasserstoff, $C_1$-$C_8$-Alkyl oder $C_1$-$C_3$-Alkoxy oder
$R_{10}$ und $R_{11}$ gemeinsam mit dem Stickstoffatom, an das sie gebunden sind, einen 5- bis 6-gliedrigen Heterocyclus, welcher noch ein weiteres Heteroatom aus der Gruppe N, O und S enthalten kann,
$R_{12}$, $R_{13}$ und $R_{14}$ Wasserstoff oder einen gegebenenfalls substituierten Alkyl-, Alkenyl-, Alkinyl-, Cycloalkyl-, Phenyl- oder Naphthylrest oder einen gegebenenfalls substituierten heterocyclischen Rest oder $R_{12}$ und $R_{13}$ auch ein Kation oder $R_{14}$ auch einen Alkoxyrest und $R_{15}$ Wasserstoff, Amino, mono- oder disubstituiertes Amino oder einen gegebenenfalls substituierten Alkyl-, Alkenyl-, Cycloalkyl- oder Phenylrest oder $R_{14}$ und $R_{15}$ gemeinsam mit dem Stickstoffatom, an das sie gebunden sind, einen gegebenenfalls substituierten heterocyclischen Rest bedeuten, oder A und Z zusammen einen gegebenenfalls substituierten Tetrahydrofuran-2-on-Ring bilden, unter Einschluss ihrer Säureadditionssalze und Metallkomplexe, oder ein Mittel, welches eine dieser Verbindungen enthält, verwendet.

3. Verfahren nach Anspruch 1, dadurch gekennzeichnet, dass man eine Verbindung der Formel I, in welcher $R_1$ Wasserstoff oder Halogen, $R_2$ Wasserstoff, $R_3$ Wasserstoff, Halogen oder Nitro, $R_4$ und $R_5$ Wasserstoff und $R_6$ Wasserstoff oder $C_1$-$C_4$-Alkyl bedeuten, oder ein Mittel, welches eine dieser Verbindungen enthält, verwendet.

4. Verfahren nach Anspruch 3, dadurch gekennzeichnet, dass man eine Verbindung der Formel I, in welcher $R_1$ Wasserstoff, Chlor, Brom oder Jod, $R_2$ Wasserstoff, $R_3$ Wasserstoff, Chlor, Brom oder Nitro, $R_4$ und $R_5$ Wasserstoff und $R_6$ Wasserstoff oder Methyl bedeuten, oder ein Mittel, welches eine dieser Verbindungen enthält, verwendet.

5. Verfahren nach Anspruch 1, dadurch gekennzeichnet, dass man eine Verbindungen der Formel I, in welcher A für $-CH_2-$ steht, oder ein Mittel, welches eine dieser Verbindungen enthält, verwendet.

6. Verfahren nach Anspruch 1, dadurch gekennzeichnet, dass man eine Verbindung der Formel I, in welcher A für $-CH_2CH_2-$ steht, oder ein Mittel, welches eine dieser Verbindungen enthält, verwendet.

7. Verfahren nach Anspruch 1, dadurch gekennzeichnet, dass man eine Verbindung der Formel I, in welcher A für $-CH(CH_3)-$ steht, oder ein Mittel, welches eine dieser Verbindungen enthält, verwendet.

8. Verfahren nach Anspruch 1, dadurch gekennzeichnet, dass man eine Verbindungen der Formel I, in welcher Z für

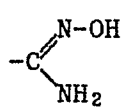

steht, oder ein Mittel, welches eine dieser Verbindungen enthält, verwendet.

9. Verfahren nach Anspruch 1, dadurch gekennzeichnet, dass man eine Verbindung der Formel I, in welcher Z für

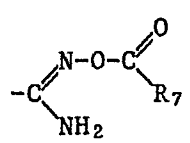

steht, worin $R_7$ unsubstituiertes oder durch Halogen mono- oder disubstituiertes oder durch $C_1$-$C_4$-Alkoxy mono-substituiertes $C_1$-$C_4$-Alkyl, oder Cyclopropyl, $C_2$-$C_3$-Alkenyl, unsubstituiertes oder durch

Halogen monosubstituiertes Phenyl, oder Benzyl, unsubstituiertes oder durch Halogen monosubstituiertes Furanyl, oder Tetrahydrofuranyl, Thienyl oder dihalogeniertes Pyrimidin bedeutet, oder ein Mittel, welches eine dieser Verbindungen enthält, verwendet.

10. Verfahren nach Anspruch 9, dadurch gekennzeichnet, dass man eine Verbindung der Formel I, in welcher $R_7$ unsubstituiertes oder durch Chlor oder Brom mono- oder disubstituiertes oder durch $C_1$-$C_4$-Alkoxy monosubstituiertes $C_1$-$C_4$-Alkyl, oder Cyclopropyl, $C_2$-$C_3$-Alkenyl, unsubstituiertes oder durch Chlor monosubstituiertes Phenyl, oder Benzyl, unsubstituiertes oder durch Brom monosubstituiertes Furanyl, oder Tetrahydrofuranyl, Thienyl oder Dichlorpyrimidin bedeutet, oder ein Mittel, welches eine dieser Verbindungen enthält, verwendet.

11. Verfahren nach Anspruch 1, dadurch gekennzeichnet, dass man eine Verbindung der Formel I, in welcher Z für

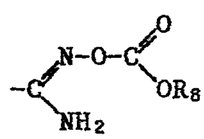

steht, worin $R_8$ unsubstituiertes oder durch Halogen monosubstituiertes $C_1$-$C_4$-Alkyl, oder Allyl, Phenyl oder Benzyl bedeutet, oder ein Mittel, welches eine dieser Verbindungen enthält, verwendet.

12. Verfahren nach Anspruch 11, dadurch gekennzeichnet, dass man eine Verbindung der Formel I, in welcher $R_8$ unsubstituiertes oder durch Brom monosubstituiertes $C_1$-$C_4$-Alkyl, oder Allyl, Phenyl oder Benzyl bedeutet, oder ein Mittel, welches eine dieser Verbindungen enthält, verwendet.

13. Verfahren nach Anspruch 1, dadurch gekennzeichnet, dass man eine Verbindung der Formel I, in welcher Z für

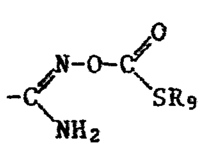

steht, worin $R_9$ für $C_1$-$C_5$-Alkyl steht, oder ein Mittel, welches eine dieser Verbindungen enthält, verwendet.

14. Verfahren nach Anspruch 1, dadurch gekennzeichnet, dass man eine Verbindungen der Formel I, in welcher Z für

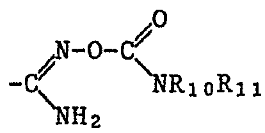

steht, worin $R_{10}$ $C_1$-$C_4$-Alkyl oder unsubstituiertes oder durch Halogen mono- oder disubstituiertes oder durch Trihalogenmethyl monosubstituiertes Phenyl und $R_{11}$ Wasserstoff oder Methoxy bedeutet, oder ein Mittel, welches eine dieser Verbindungen enthält, verwendet.

15. Verfahren nach Anspruch 14, dadurch gekennzeichnet, dass man eine Verbindung der Formel I, in welcher $R_{10}$ für $C_1$-$C_4$-Alkyl oder unsubstituiertes oder durch Chlor mono- oder disubstituiertes oder durch Trifluormethyl monosubstituiertes Phenyl und $R_{11}$ für Wasserstoff oder Methoxy steht, oder ein Mittel, welches eine dieser Verbindungen enthält, verwendet.

16. Verfahren nach Anspruch 1, dadurch gekennzeichnet, dass man eine Verbindung der Formel I, in welcher Z für $-COOR_{12}$ steht, worin $R_{12}$ Wasserstoff, ein Alkalimetallkation, das Ammoniumkation oder ein durch $C_1$-$C_4$-Alkyl oder Monohydroxy-$C_1$-$C_4$-Alkyl trisubstituiertes Amoniumkation, $C_1$-$C_{12}$-Alkyl, durch Halogen, $C_1$-$C_3$-Alkoxy, Phenoxy, Phenyl oder Tetrahydrofuranyl monosubstituiertes $C_1$-$C_4$-Alkyl, oder $C_3$-$C_6$-Alkenyl, $C_3$-$C_4$-Alkinyl, Cyclohexyl oder unsubstituiertes oder durch Methyl mono- oder disubstituiertes Phenyl bedeutet, oder ein Mittel, welches eine dieser Verbindungen enthält, verwendet.

17. Verfahren nach Anspruch 16, dadurch gekennzeichnet, dass man eine Verbindung der Formel I, in welcher $R_{12}$ für Wasserstoff, das Natriumkation, das Kaliumkation, das Ammoniumkation, ein durch $C_1$-$C_4$-Alkyl oder 2-Hydroxyethyl trisubstituiertes Ammoniumkation, $C_1$-$C_4$-Alkyl,

durch Chlor, $C_1$-$C_3$-Alkoxy, Phenoxy, Phenyl oder Tetrahydrofuranyl monosubstituiertes $C_1$-$C_4$-Alkyl, Methylallyl, 2-Propinyl, Cyclohexyl oder unsubstituiertes oder durch Methyl mono-oder disubstituiertes Phenyl steht, oder ein Mittel, welches eine dieser Verbindungen enthält, verwendet.

18. Verfahren nach Anspruch 1, dadurch gekennzeichnet, dass man eine Verbindung der Formel I, in welcher Z für $-COSR_{13}$ steht, worin $R_{13}$ $C_5$-$C_{10}$-Alkyl bedeutet, oder ein Mittel, welches eine dieser Verbindungen enthält, verwendet.

19. Verfahren nach Anspruch 18, dadurch gekennzeichnet, dass man eine Verbindung der Formel I, in welcher $R_{13}$ für n-Octyl steht, oder ein Mittel, welches eine dieser Verbindungen enthält, verwendet.

20. Verfahren nach Anspruch 1, dadurch gekennzeichnet, dass man eine Verbindung der Formel I, in welcher Z für $-CONR_{14}R_{15}$ steht, worin $R_{14}$ Wasserstoff, $C_1$-$C_{12}$-Alkyl, durch Hydroxy, $C_1$-$C_4$-Alkoxy, Di-($C_1$-$C_4$-alkyl)-amino, (Monohydroxy-$C_1$-$C_4$-alkyl)-amino, Di-(monohydroxy-$C_1$-$C_4$-alkyl)-amino, Phenyl, Tetrahydrofuranyl, Piperidinyl oder Morpholinyl monosubstituiertes $C_1$-$C_4$-Alkyl, oder Allyl, Cyclohexyl oder Amino und $R_{15}$ Wasserstoff, $C_1$-$C_4$-Alkyl oder Monohydroxy-$C_1$-$C_4$-Alkyl bedeuten oder worin $R_{14}$ und $R_{15}$ gemeinsam mit dem Stickstoffatom, an welches sie gebunden sind, den Morpholinring bilden, oder ein Mittel, welches eine dieser Verbindungen enthält, verwendet.

21. Verfahren nach Anspruch 20, dadurch gekennzeichnet, dass man eine Verbindung der Formel I, in welcher Z für $-CONR_{14}R_{15}$ steht, worin $R_{14}$ Wasserstoff, $C_1$-$C_4$-Alkyl, durch Hydroxy, $C_1$-$C_4$-Alkoxy, Di-($C_1$-$C_4$-alkyl)-amino, (Monohydroxy-$C_1$-$C_4$-alkyl)-amino, Di-(monohydroxy-$C_1$-$C_4$-alkyl)-amino, Phenyl, Tetrahydrofuranyl, Piperidinyl oder Morpholinyl monosubstituiertes $C_1$-$C_4$-Alkyl, oder Allyl, Cyclohexyl oder Amino und $R_{15}$ Wasserstoff, $C_1$-$C_4$-Alkyl oder Monohydroxy-$C_1$-$C_4$-Alkyl bedeuten oder worin $R_{14}$ und $R_{15}$ gemeinsam mit dem Stickstoffatom, an das sie gebunden sind, den Morpholinring bilden, oder ein Mittel, welches eine dieser Verbindungen enthält, verwendet.

22. Verfahren nach Anspruch 1, dadurch gekennzeichnet, dass man eine Verbindung der Formel I, in welcher A und Z gemeinsam für Tetrahydrofuran-2-on stehen, oder ein Mittel, welches eine dieser Verbindungen enthält, verwendet.

23. Verfahren nach Anspruch 1, dadurch gekennzeichnet, dass man eine Verbindung der Formel I, in welcher $R_1$ Wasserstoff, Chlor, Brom oder Jod, $R_2$ Wasserstoff, $R_3$ Wasserstoff, Chlor, Brom oder Nitro, $R_4$ und $R_5$ Wasserstoff, $R_6$ Wasserstoff oder Methyl, A eine der Gruppen $-CH_2-$, $-CH_2CH_2-$ oder $-CH(CH_3)-$, und Z Cyan,

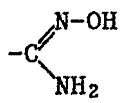 , 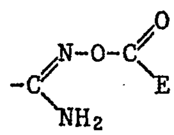 ,

$-COOR_{12}$, $-COSR_{13}$ oder $-CONR_{14}R_{15}$ bedeuten, worin E $-R_7$, $-OR_8$, $-SR_9$ oder $-NR_{10}R_{11}$ bedeutet und
$R_7$ für unsubstituiertes oder durch Chlor oder Brom mono- oder disubstituiertes oder durch $C_1$-$C_4$-Alkoxy monosubstituiertes $C_1$-$C_4$-Alkyl, oder für Cyclopropyl, $C_2$-$C_3$-Alkenyl, unsubstituiertes oder durch Chlor monosubstituiertes Phenyl, oder für Benzyl, unsubstituiertes oder durch Brom monosubstituiertes Furanyl, oder für Tetrahydrofuranyl, Thienyl oder Dichlorpyrimidin steht,
$R_8$ für unsubstituiertes oder durch Brom monosubstituiertes $C_1$-$C_4$-Alkyl, oder für Allyl, Phenyl oder Benzyl steht,
$R_9$ für $C_1$-$C_5$-Alkyl steht,
$R_{10}$ für $C_1$-$C_4$-Alkyl oder unsubstituiertes oder durch Chlor mono- oder disubstituiertes oder durch Trifluormethyl mono-substituiertes Phenyl steht,
$R_{11}$ für Wasserstoff oder Methoxy steht,
$R_{12}$ Wasserstoff, das Natriumkation, das Kaliumkation, das Ammoniumkation, durch $C_1$-$C_4$-Alkyl oder 2-Hydroxyethyl trisubstituiertes Ammoniumkation, $C_1$-$C_4$-Alkyl, durch Chlor, $C_1$-$C_3$-Alkoxy, Phenoxy, Phenyl oder Tetrahydrofuranyl monosubstituiertes $C_1$-$C_4$-Alkyl, oder Methyllallyl, 2-Propinyl, Cyclohexyl oder unsubstituiertes oder durch Methyl mono- oder disubstituiertes Phenyl bedeutet,

$R_{13}$ n-Octyl bedeutet,
$R_{14}$ Wasserstoff, $C_1$-$C_4$-Alkyl, durch Hydroxy, $C_1$-$C_4$-Alkoxy, Di-($C_1$-$C_4$-alkyl)-amino, (Monohydroxy-$C_1$-$C_4$-alkyl)-amino, Di-(monohydroxy-$C_1$-$C_4$-aklyl)-amino, Phenyl, Tetrahydrofuranyl, Piperidinyl oder Morpholinyl monosubstituiertes $C_1$-$C_4$-Alkyl, oder Allyl, Cyclohexyl oder Amino bedeutet, und
$R_{15}$ Wasserstoff, $C_1$-$C_4$-Alkyl oder Monohydroxy-$C_1$-$C_4$-Alkyl bedeutet, oder
$R_{14}$ und $R_{15}$ gemeinsam mit dem Stickstoffatom, an welches sie gebunden sind, den Morpholinring bilden, oder worin A und Z gemeinsam für Tetrahydrofuran-2-on stehen,
oder ein Mittel, welches eine dieser Verbindungen enthält, verwendet.

24. Verfahren nach Anspruch 1, dadurch gekennzeichnet, dass man eine Verbindung der Formel I, in welcher $R_1$, $R_2$, $R_4$, $R_5$ und $R_6$ Wasserstoff, $R_3$ Wasserstoff oder Chlor, A die Gruppe $-CH_2-$ oder $-CH(CH_3)-$ und Z eine Carbonsäureestergruppe $-COOR_{12}$, worin $R_{12}$ für $C_1$-$C_{12}$-Alkyl oder durch Phenyl monosubstituiertes $C_1$-$C_4$-Alkyl oder für $C_3$-$C_6$-Alkenyl steht, bedeuten, oder ein Mittel, welches eine dieser Verbindungen enthält, verwendet.

25. Verfahren nach Anspruch 1, dadurch gekennzeichnet, dass man O-(Methoxycarbonyl)-2-(8-chinolinoxy)-acetamidoxim oder ein Mittel, welches diese Verbindung enthält, verwendet.

26. Verfahren nach Anspruch 1, dadurch gekennzeichnet, dass man 2-(5-Chlor-8-chinolinoxy)-essigsäure-methylallylester oder ein Mittel, welches diese Verbindung enthält, verwendet.

27. Verfahren nach Anspruch 1 zum Schützen von Kulturpflanzen gegen schädigende Wirkungen von Herbiziden der Formel II, worin M für -OR' steht und R' die für Formel II angegebene Bedeutung hat und Q für 6-Halogenbenzoxazol-2-yl, 6-Halogenbenzothiazol-2-yl oder 6-Halogen-chinoxalin-2-yl steht.

28. Verfahren nach Anspruch 1 zum Schützen von Kulturpflanzen gegen schädigende Wirkungen von 2-[4-(6-Chlorbenzoxazol-2-yl-oxy)-phenoxy]-propionsäure-ethylester.

29. Verfahren nach Anspruch 1 zum Schützen von Kulturpflanzen gegen schädigende Wirkungen von 2-[4-(6-Chlorbenzothiazol-2-yl-oxy)-phenoxy]-propionsäure-ethylester.

30. Verfahren nach Anspruch 1 zum Schützen von Kulturpflanzen gegen schädigende Wirkungen von 2-[4-(6-Chlorchinoxalin-2-yl-oxy)-phenoxy]-propionsäure-ethylester.

31. Verfahren nach Anspruch 1 zum Schützen von Kulturpflanzen gegen schädigende Wirkungen von 2-[4-(6-Chlorbenzoxazol-2-yl-oxy)-phenoxy]-propionsäure-ethylester, 2-[4-(6-Chlorbenzothiazol-2-yl-oxy)-phenoxy]-propionsäure-ethylester oder 2-[4-(6-Chlorchinoxalin-2-yl-oxy)-phenoxy]-propionsäure-ethylester, dadurch gekennzeichnet, dass man die Kulturpflanzen, Teile dieser Pflanzen oder für den Anbau der Kulturpflanzen bestimmte Böden mit O-(Methoxycarbonyl)-2-(8-chinolinoxy)-acetamidoxim, 2-(5-Chlor-8-chinolinoxy)-essigsäure-methylallylester oder einem Mittel, welches eine dieser Verbindungen enthält, behandelt.

32. Verfahren nach Anspruch 1 zum Schützen von Getreide.

33. Verfahren nach Anspruch 32 zum Schützen von Weizen, Gerste und Mais.

34. Verfahren nach Anspruch 1, dadurch gekennzeichnet, dass man Kulturpflanzenbestände oder Anbauflächen für Kulturpflanzen mit 0,1 bis 10 kg/ha einer Verbindung der Formel I gemäss Anspruch 1 behandelt.

35. Verfahren nach Anspruch 34, dadurch gekennzeichnet, dass man Kulturpflanzenbestände oder Anbauflächen für Kulturpflanzen mit 0,5 bis 2 kg/ha einer Verbindung der Formel I gemäss Anspruch 1 behandelt.

36. Verfahren nach Anspruch 1, dadurch gekennzeichnet, dass man Samen der Kulturpflanzen mit einer Verbindung der Formel I gemäss Anspruch 1 behandelt.

37. Verfahren nach Anspruch 36, dadurch gekennzeichnet, dass man Samen der Kulturpflanze mit 0,01 bis 10 g/kg Samen einer Verbindung der Formel I gemäss Anspruch 1 behandelt.

38. Verfahren nach Anspruch 37, dadurch gekennzeichnet, dass man Samen der Kulturpflanzen mit 0,05 bis 1 g/kg Samen einer Verbindung der Formel I gemäss Anspruch 1 behandelt.

39. Verfahren nach Anspruch 36 zum Schützen von Kulturpflanzen gegen schädigende Wirkungen von 2-[4-(6-Chlorbenzoxazol-2-yl-oxy)-phenoxy]-propionsäure-ethylester, 2-[4-(6-Chlorbenzothiazol-2-yl-oxy)-phenoxy]-propionsäure-ethylester oder 2-[4-(6-Chlorchinoxalin-2-yl-oxy)-phenoxy]-propionsäure-ethylester, dadurch gekennzeichnet, dass man die Samen der Kulturpflanzen mit O-(Methoxycarbonyl)-2-(8-chinolinoxy)-acetamidoxim oder 2-(5-Chlor-8-chinolinoxy)-essigsäure-methylallylester behandelt.

40. Verfahren nach Anspruch 39 zum Schützen von Getreide.

41. Verwendung von Verbindungen der Formel I gemäss Anspruch 1 zum Schützen von Kulturpflanzen gegen schädigende Wirkungen von Verbindungen der Formel II gemäss Anspruch 1.

42. Verfahren zur selektiven Bekämpfung von Unkräutern in Kulturpflanzenbeständen, dadurch gekennzeichnet, dass man die Kulturpflanzenbestände, Teile der Kulturpflanzen oder Anbauflächen der Kulturpflanzen mit einem Antidot der Formel I gemäss Anspruch 1 und einem Herbizid der Formel II gemäss Anspruch 1 behandelt.

FO 7.5/SES/we*

Europäisches Patentamt

# EUROPÄISCHER RECHERCHENBERICHT

0159290
Nummer der Anmeldung

EP 85 81 0125

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | EP-A-0 086 750 (CIBA-GEIGY)<br>* Seite 7, Zeile 21 - Seite 11, Zeile 8; Seite 12, Zeilen 1-27; Seite 17, Zeilen 20-26; Ansprüche * | 1-15, 23,25, 27-42 | A 01 N 25/32 |
| D,X | EP-A-0 094 349 (CIBA-GEIGY)<br>* Seite 23, Zeile 13 - Seite 27, Zeile 10; Seite 28, Zeilen 6-28; Seite 33, Zeile 24 - Seite 34, Zeile 4; Ansprüche * | 1-7,16 -24,26 -42 | |
| D,A | DE-A-2 640 730 (HOECHST)<br>* Ansprüche; Seite 12, Zeile 25 - Seite 13, Zeile 6 * | 1-42 | RECHERCHIERTE SACHGEBIETE (Int. Cl.4) |
| D,A | DE-A-3 004 770 (NISSAN CHEMICAL INDUSTRIES)<br>* Ansprüche; Seite 37, Zeile 4 - Seite 38, Zeile 6 * | 1-42 | A 01 N |
| A | DE-A-2 546 845 (BASF)<br>* Ansprüche; Seite 11, Zeile 8 - Seite 14, Zeile 8; Seite 19, Zeilen 10-19 * | 1-7,16 ,17,20 ,21,23 ,24,26 -42 | |

Der vorliegende Recherchenbericht wurde für alle Patentansprüche erstellt.

| Recherchenort | Abschlußdatum der Recherche | Prüfer |
|---|---|---|
| DEN HAAG | 24-06-1985 | FLETCHER A.S. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82